# EUROPÄISCHE PATENTSCHRIFT

(11) **EP 4 445 921 B1**
(45) Veröffentlichungstag und Bekanntmachung des Hinweises auf die Patenterteilung: **11.06.2025**
(21) Anmeldenummer: 24192002.4
(22) Anmeldetag: 26.07.2022
(51) Int. Cl.: A61L 2/10, A61L 2/20, A61L 2/24

(54) **VERFAHREN ZUM EINSCHLEUSEN VON ARTIKELN AUS EINEM GEBINDE IN DIE PROZESSKAMMER EINES CONTAINMENTS UNTER ASEPTISCHEN BEDINGUNGEN UND SCHLEUSENANORDNUNG DAZU**
METHOD FOR INTRODUCING ARTICLES FROM A CONTAINER INTO THE PROCESS CHAMBERS OF A CONTAINER UNDER ASEPTIC CONDITIONS AND LOCK ARRANGEMENT THEREFOR
PROCÉDÉ POUR INTRODUIRE DES ARTICLES D'UN EMBALLAGE DANS LA CHAMBRE DE TRAITEMENT D'UNE ENCEINTE DE CONFINEMENT DANS DES CONDITIONS ASEPTIQUES ET DISPOSITIF DE SAS

(43) Veröffentlichungstag der Anmeldung: 16.10.2024
(62) Teilanmeldung aus: 22187101.5
(73) Patentinhaber: SKAN AG, 4123 Allschwil (CH)
(72) Erfinder: Carli, Julia Annette, 4053 Basel (CH); Leuenberger, Philipp, 4052 Basel (CH); Müller, Mathieu, 68510 Sierentz (FR); Rethoret, Christophe, 68500 Issenheim (FR); Schneidler, Tobias Jan, 6332 Hagendorn (CH); Tondera, Marc, 4125 Riehen (CH); Zeller, Mike, 4246 Wahlen (CH)
(74) Vertreter: Maucher Jenkins Patent- und Rechtsanwälte

(56) Entgegenhaltungen:
- WO-A2-2020/016645
- BAESSLER H J ET AL: "Chapter 6 - Aseptic Transfer Systems Into and Out of Barrier Isolators and RABS", 1 January 2013 (2013-01-01), XP009512843, ISBN: 978-3-642-39291-7, Retrieved from the Internet <URL:https://www.springer.com/gp/book/9783642392917> [retrieved on 20230109]

## Beschreibung

### Anwendungsgebiet der Erfindung

Die vorliegende Erfindung betrifft ein Verfahren und eine in einem Aufstellraum positionierte Schleusenanordnung zum Einschleusen von Artikeln unter aseptischen Bedingungen aus seriell zugeführten Gebinden in eine von einem Gehäuse umgebene Prozesskammer eines Containments. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist. Die im Innenraum gelagerten Artikel sollen nach dem Öffnen der Abdeckung in der Prozesskammer behandelt werden. Die Behältnisse der Gebinde haben die Gestalt wannenförmiger Tubs, und die Artikel sind z.B. pharmazeutische Phiolen, Vials oder Spritzen, die in systematisch angeordneten muldenförmigen Aufnahmekonturen von Nestern stecken.

### Stand der Technik

Aus der WO 2020/016 645 A2 ist eine Anordnung zum kontaminationsfreien Einschleusen eines sterilen Objektes aus einem Behältnis, welches mit einer semipermeablen Abdeckung verschlossen ist, in eine von einer Wandung umgebenen Arbeitskammer eines Containments bekannt. Die Anordnung umfasst zunächst eine Toreinheit mit einem in der Wandung angeordneten Zugangsflansch, der einen Durchgang von aussen in die Arbeitskammer ausbildet, und einer den Durchgang dicht verschliessenden Tür, die sich zur Offenstellung in die Arbeitskammer bewegen lässt. Zur Anordnung gehört ferner eine Behältnisaufnahme mit einem Auffang zur Halterung eines in die Behältnisaufnahme eingebrachten Behältnisses, einer Öffnung zum Einbringen des Behältnisses in den Auffang und einem Flansch zum Zusammenwirken mit dem Zugangsflansch.

Eine Dekontaminationseinheit ist dazu vorgesehen, bei geschlossener Tür, am Zugangsflansch angedockter Behältnisaufnahme und in der Behältnisaufnahme gelagertem Behältnis, eine der Tür zugewandte Aussenfläche der Abdeckung zu dekontaminieren. In Schliessstellung ist die Tür vonseiten der Arbeitskammer abgedichtet an den Zugangsflansch angefügt, und der Zugangsflansch umrandet einen Zwischenraum, der im Durchgang liegt. Die Abdeckung des in der Behältnisaufnahme eingestellten Behältnisses ist bei an den Zugangsflansch herangeschwenkter Behältnisaufnahme zwischen dem Flansch und dem Zugangsflansch abgedichtet. Die Dekontaminationseinheit ist mit Wirkungsrichtung in den Durchgang hinein direkt an der Tür oder seitlich der Toreinheit installiert, welche die Tür und den Zugangsflansch aufweist. Die Dekontaminationseinheit ist als Strahlungsquelle, z.B. UVC, UV, E-Beam oder Puls-Licht, oder als Begasungseinrichtung, z.B zur Verneblung einer H2 O2 -Lösung ausgebildet.

Aus Baessler H.-J. et al, 2013, "Chapter 6 - Aseptic Transfer Systems Into and Out of Barrier Isolators und RABS", Containment Technology, Springer Verlag, Berlin-Heidelberg, S. 81 - 104 sind Verfahren zum Transfer von Artikeln in und aus Isolatoren bekannt.

### Aufgabe der Erfindung

Angesichts der beschränkten Produktivität der gemäss vorbekanntem Stand der Technik existierenden Vorrichtungen und praktizierten Verfahren, liegt der Erfindung die Aufgabe zugrunde, ein für das hiesige Anwendungsgebiet effizienteres Verfahren zum Einschleusen von Artikeln aus einem Gebinde in die Prozesskammer eines Containments unter aseptischen Bedingungen und eine dazu geeignete Schleusenanordnung vorzuschlagen. Massgeblich hierbei sind ein möglichst hoher Automatisierungsgrad, die weitgehende Vermeidung manuellen Eingreifens und Gewährleistung hoher Reinheitserfordernisse. Ausserdem ist darauf zu orientieren, dass bei möglichst geringem zeitlichem und apparativem Dekontaminationsaufwand möglichst nur Artikel in die Prozesskammer transferiert werden, die zu verarbeiten sind.

### Übersicht über die Erfindung

Zur Lösung der genannten Aufgabe sind erfindungsgemäß bei einem Verfahren die Merkmale des Anspruchs 1 und bei einer Transfervorrichtung die Merkmale des Anspruchs 4 vorgesehen. Vorteilhafte Ausgestaltungen ergeben sich durch die Unteransprüche.

Das erfindungsgemässe Verfahren bzw. die erfindungsgemässe Schleusenanordnung ist zum Einschleusen von Artikeln unter aseptischen Bedingungen aus seriell zugeführten Gebinden in eine von einem Gehäuse umgebene Prozesskammer eines Containments mittels einer in einem Aufstellraum positionierten Schleusenanordnung konzipiert. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist, wobei die im Innenraum gelagerten Artikel nach dem Öffnen der Abdeckung in der Prozesskammer zu behandeln sind.

Im Produktionsmodus der Schleusenanordnung werden die aufeinanderfolgenden Verfahrensschritte ausgeführt:
- zum Transfer der Artikel aus den Gebinden in die Prozesskammer durch von der Eingangsstation sind zumindest zwei in die Prozesskammer führende Passagen vorgesehen und jede der Passagen wird einerseits von einem in der Prozesskammer herangeführten Deckel und/oder andererseits von einem in der Eingangsstation herangeführten Gebinde mit dessen der Passage zugewandten Abdeckung verschlossen;
- bei an die Passage herangeführtem Gebinde wird die der Passage zugewandte Oberfläche der Abdeckung mit eingeschlossenem Umfeld mittels einer Dekontaminationsvorrichtung dekontaminiert;
- nach erfolgter Dekontamination der der Passage zugewandten Oberfläche der Abdeckung, einschliesslich deren Umfeld, wird die Abdeckung geöffnet, um die Artikel aus dem Behältnis durch dessen Durchlass in die Prozesskammer zu transferieren; und
- nach Entleerung des Behältnisses wird zunächst der Deckel wieder an die betreffende Passage herangebracht, um anschliessend das von den Artikeln geleerte Behältnis von der Passage zu entfernen; wobei
- der Deckel an den Passagen im Wechsel zum Einsatz kommt.

Nachfolgend werden spezielle Ausführungsformen des erfindungsgemässen Verfahrens bzw. der Schleusenanordnung definiert:
Die Dekontaminationsvorrichtung ist im Deckel integriert oder ausserhalb des Deckels angeordnet, wobei das Emissionselement der Dekontaminationsvorrichtung direkt im Deckel oder ausserhalb des Deckels in einer Aufnahme sitzt.

Das eingeschlossene Umfeld umfasst das darin vorhandene Gasvolumen und vom Gasvolumen umspülte Oberflächen, nämlich Oberflächen:
- des Deckels;
- der optionalen Aufnahme;
- einer optionalen Dichtung; und
- von optional freiliegenden Wandungsflächen.

Die Prozesskammer ist mit einem Manipulator oder zusätzlich mit einem zweiten Manipulator ausgestattet, an deren schwenkbaren Auslegern jeweils ein mit Werkzeugen bestückter Werkzeugkopf sitzt, wobei:
- die Werkzeuge ausgebildet sind, um die Abdeckung zu öffnen und anschliessend die Artikel aus dem Behältnis durch den freigelegten Durchlass in die Prozesskammer zu transferieren; und
- der eine Manipulator mit dessen Werkzeugen wechselhaft an den Passagen zum Öffnen der Abdeckung und Transfer der Artikel in die Prozesskammer zum Einsatz kommt; oder
- der eine Manipulator und der zweite Manipulator jeweils nur an einer der Passagen oder die beiden Manipulatoren wechselhaft an den Passagen zum Einsatz kommen.

Ein Schneidwerkzeug ist Komponente der Werkzeuge, um für das Öffnen die Abdeckung aufzuschneiden. Vorzugsweise bestehen von der Abdeckung und der Umhüllung zumindest Flächenanteile aus einem semipermeablen Vliesgewebe, wie Tyvek^{®} . Die Abdeckung bewahrt vor dem Entfernen den Innenraum des Behältnisses in sterilem Zustand und ist auf einem den Durchlass des Behältnisses umlaufenden Rand versiegelt, z.B. durch Verkleben oder Verschweissen.

Im Behältnis lagert zur Aufnahme einer Vielzahl von Artikeln ein entnehmbares Nest, welches zusammen mit den Artikeln in die Prozesskammer transferiert wird. Nach Leerung des Nestes von den Artikeln in der Prozesskammer wird dieses geleerte Nest aus der Prozesskammer in ein nächstes oder dasselbe an der betreffenden Passage angedrücktes geleertes Behältnis entsorgt. Alternativ werden die Artikel direkt aus dem im Behältnis verbleibenden Nest in die Prozesskammer transferiert.

Die Dekontaminationsvorrichtung wird vorzugsweise als Strahlungsquelle, z.B. UVC, UV, E-Beam oder Puls-Licht, oder alternativ als eine Begasungseinrichtung, **z.B.** zur Verneblung einer H2 O2 -Lösung, ausgebildet.

Die von einem Gehäuse umgebene Eingangsstation weist einen zu einer angrenzenden Bereitstellungsstation führenden Zugang auf, wobei:
- die Eingangsstation mit einer gereinigten, gleichgerichteten Verdrängungsströmung betrieben wird, die teilweise durch den Zugang austritt;
- an der Bereitstellungsstation die Gebinde angeliefert werden, welche im Anlieferungszustand jeweils von einer Umhüllung umgeben sind, um das Innenvolumen des Gebindes mit dessen Inhalt aseptisch zu erhalten; und
- an der Bereitstellungsstation eine an der Umhüllung angebrachte Öffnung, die man bis anhin geschlossen hält, nun auf der der aus dem Zugang austretenden Verdrängungsströmung zugewandten Seite freigegeben wird, um das einzelne Gebinde in die Eingangsstation einzubringen; oder
- an der Bereitstellungsstation die Umhüllung auf der der aus dem Zugang austretenden Verdrängungsströmung zugewandten Seite aufgetrennt wird, um das einzelne Gebinde in die Eingangsstation einzubringen.

Die Positionierung des in der zuerst noch geschlossenen Umhüllung steckenden Gebindes erfolgt auf einer Auflage. Zum Ausschieben des Gebindes aus der offenen Umhüllung, bei auf das Gebinde fliessender Verdrängungsströmung, dient ein Vorschubmittel. Die Umhüllung wird von einem Greifer, vorzugsweise als Sauger ausgebildet, gehalten.

Die in die Eingangsstation eingebrachten Gebinde werden mittels zumindest einer Fördereinrichtung den Passagen zugeführt und nach der Verarbeitung über diese Fördereinrichtung wieder abgeführt. Die zumindest eine Fördereinrichtung beruht vorzugsweise auf dem Prinzip der Magnetschwebetechnik und erlaubt die Förderung auf mehreren Spuren, auch in gegensätzlicher Richtung.

Mittels einer ersten Fördereinrichtung erfolgt der Transport der Gebinde innerhalb der Eingangsstation, während eine zweite Fördereinrichtung das Heranschwenken der Gebinde mit an der jeweiligen Passage angepressten Abdeckung bewirkt. Die zweite Fördereinrichtung ist auch zum Entfernen bearbeiteter Gebinde von der jeweiligen Passage nutzbar. Zur Positionierung der Gebinde an der jeweiligen Passage umfasst die zweite Fördereinrichtung vorzugsweise ein kardanisches Lager.

Die zweite Fördereinrichtung besteht aus:
- einer Achse, durch welche sich eine erste Drehachse erstreckt;
- zwei nebeneinander an der Achse fixierten Gabeln, die jeweils um eine zweite Drehachse schwenkbar sind, welche sich rechtwinklig zur ersten Drehachse erstrecken; und
- jeweils einen in der zugehörigen Gabel aufgehängten und um eine dritte Drehachse schwenkbaren Träger, wobei die erste Drehachse und die dritte Drehachse zueinander parallel verlaufen und die Träger zum Unterfassen der Ränder der Behältnisse beim An- bzw. Abschwenken von den Passagen konfiguriert sind.

Zwei nebeneinander liegende kardanische Lager sind jeweils gebildet aus einen U-förmigen Träger, der in der dritten Drehachse jeweils mit einer Gabel verbunden ist, die in der zweiten Drehachse aufgehängt ist.

In jeder Passage sitzt eine Dichtung, welche zusammen mit der Abdeckung eines temporär angepressten Gebindes und/oder mit einem temporär angepressten Deckel an den Passagen, im Übergang von der Eingangsstation zur Prozesskammer, Abdichtungen schafft. Anstelle einer herkömmlichen Dichtung kann man einen minimen Spalt vorsehen oder eine LabyrinthDichtung einsetzen. Der Deckel wird von einem schwenkbaren Ausleger einer in der Prozesskammer positionierten Apparatur getragen.

Der zumindest eine Manipulator oder auch der zusätzliche Manipulator sind vorzugsweise als Roboter ausgebildet.

Die Behältnisse der Gebinde haben die Gestalt wannenförmiger Tubs. Die Artikel sind z.B. Phiolen, Vials oder Spritzen, die in systematisch angeordneten Aufnahmekonturen von Nestern stecken.

Das Wesen der Erfindungen beruht auf dem unabhängigen Verfahrensanspruch 1 bzw. auf dem unabhängigen Sachanspruch 18.

Bei einer ersten Ausgestaltung der Erfindung kann bei einem Verfahren nach Anspruch 10, vorgesehen sein, dass
a) die Dekontaminationsvorrichtung (7) im Deckel (51) integriert oder ausserhalb des Deckels (51) angeordnet ist; und
b) das Emissionselement der Dekontaminationsvorrichtung (7) direkt im Deckel (51) oder ausserhalb des Deckels (51) in einer Aufnahme (70) angeordnet ist.

Bei einer zweiten Ausgestaltung der Erfindung kann bei einem Verfahren nach Anspruch 10 oder bei einer vorstehenden Ausgestaltung vorgesehen sein, dass das eingeschlossene Umfeld (8) das darin vorhandene Gasvolumen und vom Gasvolumen umspülte Oberflächen umfasst, nämlich Oberflächen:
a) des Deckels (51);
b) der optionalen Aufnahme (70);
c) einer optionalen Dichtung (470,480); und
d) von optional freiliegenden Wandungsflächen.

Bei einer dritten Ausgestaltung der Erfindung kann bei einem Verfahren nach Anspruch 10 oder bei einer vorstehenden Ausgestaltung vorgesehen sein, dass
a) die Prozesskammer (43) mit einem Manipulator (6) oder zusätzlich mit einem zweiten Manipulator (6) ausgestattet ist, an deren schwenkbaren Auslegern (60) jeweils ein mit Werkzeugen bestückter Werkzeugkopf (61) sitzt, wobei:
b) die Werkzeuge ausgebildet sind, um die Abdeckung (98) zu öffnen und anschliessend die Artikel (960) aus dem Behältnis (94) durch den freigelegten Durchlass (95) in die Prozesskammer (43) zu transferieren; und
c) der eine Manipulator (6) mit dessen Werkzeugen wechselhaft an den Passagen (47,48) zum Öffnen der Abdeckung (98) und Transfer der Artikel (960) in die Prozesskammer (43) zum Einsatz kommt; oder
d) der eine Manipulator (6) und der zweite Manipulator (6) jeweils nur an einer der Passagen (47,48) oder die beiden Manipulatoren (6) wechselhaft an den Passagen (47,48) zum Einsatz kommen.

Bei einer vierten Ausgestaltung der Erfindung kann bei einem Verfahren nach Anspruch 10 oder bei einer vorstehenden Ausgestaltung vorgesehen sein, dass
a) ein Schneidwerkzeug Komponente der Werkzeuge ist, um für das Öffnen die Abdeckung (98) aufzuschneiden; wobei
b) vorzugsweise von der Abdeckung (98) und der Umhüllung (99) zumindest Flächenanteile aus einem semipermeablen Vliesgewebe, wie Tyvek ^{®} bestehen, und die Abdeckung (98) vor dem Entfernen den Innenraum (940) des Behältnisses (94) in sterilem Zustand bewahrt und auf einem den Durchlass (95) des Behältnisses (94) umlaufenden Rand (941) versiegelt ist.

Bei einer fünften Ausgestaltung der Erfindung kann bei einem Verfahren nach Anspruch 10 oder bei einer vorstehenden Ausgestaltung vorgesehen sein, dass
a) im Behältnis (94) zur Aufnahme einer Vielzahl von Artikeln (960) ein entnehmbares Nest (96) lagert, welches zusammen mit den Artikeln (960) in die Prozesskammer (43) transferiert wird; und
b) nach Leerung des Nestes (96) von den Artikeln (960) in der Prozesskammer (43), dieses geleerte Nest (96) aus der Prozesskammer (43) in ein nächstes oder dasselbe an der betreffenden Passage (47,48) angedrücktes geleertes Behältnis (94) entsorgt wird; oder
c) die Artikel (960) direkt aus dem im Behältnis (94) verbleibenden Nest (96) in die Prozesskammer (43) transferiert werden.

Bei einer sechsten Ausgestaltung der Erfindung kann bei einem Verfahren nach Anspruch 10 oder bei einer vorstehenden Ausgestaltung vorgesehen sein, dass die Dekontaminationsvorrichtung vorzugsweise als Strahlungsquelle, **z.B.** UVC, UV, E-Beam oder Puls-Licht, oder alternativ als eine Begasungseinrichtung, z.B zur Verneblung einer H 2 O 2 - Lösung, ausgebildet wird.

Bei einer siebten Ausgestaltung der Erfindung kann bei einem Verfahren nach Anspruch 10 oder bei einer vorstehenden Ausgestaltung vorgesehen sein, dass der Eingangsstation (3), welche von einem Gehäuse (39) umgeben ist, das einen zu einer angrenzenden Bereitstellungsstation (2) führenden Zugang (37) aufweist, wobei:
a) die Eingangsstation (3) mit einer gereinigten, gleichgerichteten Verdrängungsströmung (LF) betrieben wird, die teilweise durch den Zugang (37) austritt;
b) an der Bereitstellungsstation (2) die Gebinde (9;91,92,93) angeliefert werden, welche im Anlieferungszustand jeweils von einer Umhüllung (99) umgeben sind, um das Innenvolumen (90) des Gebindes (9;91,92,93) mit dessen Inhalt aseptisch zu erhalten; und
c) an der Bereitstellungsstation (2) eine an der Umhüllung (99) angebrachte Öffnung, die man bis anhin geschlossen hält, nun auf der der aus dem Zugang (37) austretenden Verdrängungsströmung (LF) zugewandten Seite freigegeben wird, um das einzelne Gebinde (9;91,92,93) in die Eingangsstation (3) einzubringen; oder
d) an der Bereitstellungsstation (2) die Umhüllung (99) auf der der aus dem Zugang (37) austretenden Verdrängungsströmung (LF) zugewandten Seite aufgetrennt wird, um das einzelne Gebinde (9;91,92,93) in die Eingangsstation (3) einzubringen.

Bei einer achten Ausgestaltung kann bei einem Verfahren gemäß der siebten Ausgestaltung vorgesehen sein, dass
a) die Positionierung des in der zuerst noch geschlossenen Umhüllung (99) steckenden Gebindes (9;91,92,93) auf einer Auflage (20) erfolgt
b) zum Ausschieben des Gebindes (9;91,92,93) aus der offenen Umhüllung (99), bei auf das Gebinde (9;91,92,93) fliessender Verdrängungsströmung (LF), ein Vorschubmittel (22) dient; und
c) die Umhüllung (99) von einem Greifer (21), vorzugsweise als Sauger ausgebildet, gehalten wird.

Bei einer neunten Ausgestaltung der Erfindung kann bei einem Verfahren nach Anspruch 10 oder bei einer vorstehenden Ausgestaltung vorgesehen sein, dass
a) die in die Eingangsstation (3) eingebrachten Gebinde (9;91,92,93) mittels zumindest einer Fördereinrichtung (34,35) den Passagen (47,48) zugeführt werden; und
b) die zumindest eine Fördereinrichtung (34,35) vorzugsweise auf dem Prinzip der Magnetschwebetechnik beruht.

Bei einer zehnten Ausgestaltung der Erfindung kann bei einem Verfahren nach Anspruch 10 oder bei einer vorstehenden Ausgestaltung vorgesehen sein, dass
a) mittels einer ersten Fördereinrichtung (34) der Transport der Gebinde (9;91,92,93) innerhalb der Eingangsstation (3) erfolgt während
b) eine zweite Fördereinrichtung (35) das Heranschwenken der Gebinde (9;91,92,93) mit an der jeweiligen Passage (47,48) angepressten Abdeckung (98) bewirkt; und
c) die zweite Fördereinrichtung (35) auch zum Entfernen bearbeiteter Gebinde (9;91,92,93) von der jeweiligen Passage (47,48) nutzbar ist.

Bei einer elften Ausgestaltung der Erfindung kann bei einem Verfahren nach Anspruch 10 oder bei einer vorstehenden Ausgestaltung vorgesehen sein, dass zur Positionierung der Gebinde (9;91,92,93) an der jeweiligen Passage (47,48) die zweite Fördereinrichtung (35) ein kardanisches Lager (351) umfasst
und/oder dass die zweite Fördereinrichtung (35) besteht aus:
a) einer Achse (350), durch welche sich eine erste Drehachse (D1) erstreckt;
b) zwei nebeneinander an der Achse (350) fixierten Gabeln (352), die jeweils um eine zweite Drehachse (D2) schwenkbar sind, welche sich rechtwinklig zur ersten Drehachse (D1) erstrecken; und
c) jeweils einen in der zugehörigen Gabel (352) aufgehängten und um eine dritte Drehachse (D3) schwenkbaren Träger (353), wobei die erste Drehachse (D1) und die dritte Drehachse (D3) zueinander parallel verlaufen und die Träger (353) zum Unterfassen der Ränder (941) der Behältnisse (94) beim An- bzw. Abschwenken von den Passagen (47,48) konfiguriert sind; wobei
d) zwei nebeneinander liegende kardanische Lager (351) jeweils gebildet sind aus einen U-förmigen Träger (353), der in der dritten Drehachse (D3) jeweils mit einer Gabel (352) verbunden ist, die in der zweiten Drehachse (D2) aufgehängt ist.

Bei einer zwöften Ausgestaltung der Erfindung kann bei einem Verfahren nach Anspruch 10 oder bei einer vorstehenden Ausgestaltung vorgesehen sein, dass in jeder Passage (47,48) eine Dichtung (470,480) sitzt, welche zusammen mit der Abdeckung (98) eines temporär angepressten Gebindes (9;91,92,93) und/oder mit einem temporär angepressten Deckel (51) an den Passagen (47,48), im Übergang von der Eingangsstation (3) zur Prozesskammer (43), Abdichtungen schafft
und/oder dass der Deckel (51) von einem schwenkbaren Ausleger (50) einer in der Prozesskammer (43) positionierten Apparatur (5) getragen wird.

Bei einer dreizehnten Ausgestaltung der Erfindung kann bei einem Verfahren nach Anspruch 10 oder bei einer vorstehenden Ausgestaltung vorgesehen sein, dass der zumindest eine Manipulator (6) oder auch der zusätzliche Manipulator (6) als Roboter ausgebildet sind und/oder dass die Behältnisse (94) der Gebinde (9;91,92,93) die Gestalt wannenförmiger Tubs haben und die Artikel (960), z.B. Phiolen, Vials oder Spritzen sind, die in systematisch angeordneten Aufnahmekonturen von Nestern (96) stecken.

Bei einer ersten Ausgestaltung der Erfindung kann bei einer Schleusenanordnung nach Anspruch 11 vorgesehen sein, dass
a) die Dekontaminationsvorrichtung (7) im Deckel (51) integriert oder ausserhalb des Deckels (51) angeordnet ist; und
b) das Emissionselement der Dekontaminationsvorrichtung (7) direkt im Deckel (51) oder ausserhalb des Deckels (51) in einer Aufnahme (70) angeordnet ist.

Bei einer zweiten Ausgestaltung der Erfindung kann bei einer Schleusenanordnung nach Anspruch 11 oder bei einer vorangehenden Ausgestaltung vorgesehen sein, dass das eingeschlossene Umfeld (8) das darin vorhandene Gasvolumen und vom Gasvolumen umspülte Oberflächen umfasst, nämlich Oberflächen:
a) des Deckels (51);
b) der optionalen Aufnahme (70);
c) einer optionalen Dichtung (470,480); und
d) von optional freiliegenden Wandungsflächen.

Bei einer dritten Ausgestaltung der Erfindung kann bei einer Schleusenanordnung nach Anspruch 11 oder bei einer vorangehenden Ausgestaltung vorgesehen sein, dass
a) die Prozesskammer (43) mit einem Manipulator (6) oder zusätzlich mit einem zweiten Manipulator (6) ausgestattet ist, an deren schwenkbaren Auslegern (60) jeweils ein mit Werkzeugen bestückter Werkzeugkopf (61) sitzt, wobei:
b) die Werkzeuge ausgebildet sind, um die Abdeckung (98) zu öffnen und anschliessend die Artikel (960) aus dem Behältnis (94) durch den freigelegten Durchlass (95) in die Prozesskammer (43) zu transferieren; und
c) der eine Manipulator (6) mit dessen Werkzeugen wechselhaft an den Passagen (47,48) zum Öffnen der Abdeckung (98) und Transfer der Artikel (960) in die Prozesskammer (43) zum Einsatz kommt; oder
d) der eine Manipulator (6) und der zweite Manipulator (6) jeweils nur an einer der Passagen (47,48) oder die beiden Manipulatoren (6) wechselhaft an den Passagen (47,48) zum Einsatz kommen.

Bei einer vierten Ausgestaltung der Erfindung kann bei einer Schleusenanordnung nach Anspruch 11 oder bei einer vorangehenden Ausgestaltung vorgesehen sein, dass
a) die Werkzeuge ein Schneidwerkzeug als Komponente aufweisen, bestimmt zum Öffnen der Abdeckung (98); wobei
b) vorzugsweise von der Abdeckung (98) und der Umhüllung (99) zumindest Flächenanteile aus einem semipermeablen Vliesgewebe, wie Tyvek ^{®} bestehen, und die Abdeckung (98) vor dem Entfernen den Innenraum (940) des Behältnisses (94) in sterilem Zustand bewahrt und auf einem den Durchlass (95) des Behältnisses (94) umlaufenden Rand (941) versiegelt ist.

Bei einer fünften Ausgestaltung der Erfindung kann bei einer Schleusenanordnung nach Anspruch 11 oder bei einer vorangehenden Ausgestaltung vorgesehen sein, dass
a) im Behältnis (94) zur Aufnahme einer Vielzahl von Artikeln (960) ein entnehmbares Nest (96) lagert, welches zusammen mit den Artikeln (960) in die Prozesskammer (43) transferierbar ist; und
b) das von Artikeln (960) geleerte Nest (96) aus der Prozesskammer (43) in ein nächstes oder dasselbe an der betreffenden Passage (47,48) angedocktes geleertes Behältnis (94) entsorgbar ist; oder
c) die Artikel (960) direkt aus dem im Behältnis (94) verbleibenden Nest (96) in die Prozesskammer (43) transferierbar sind.

Bei einer sechsten Ausgestaltung der Erfindung kann bei einer Schleusenanordnung nach Anspruch 11 oder bei einer vorangehenden Ausgestaltung vorgesehen sein, dass die Dekontaminationsvorrichtung vorzugsweise als Strahlungsquelle, **z.B.** UVC, UV, E-Beam oder Puls-Licht, oder alternativ als eine Begasungseinrichtung, **z.B.** zur Verneblung einer H 2 O 2 - Lösung, ausgebildet ist.

Bei einer siebten Ausgestaltung der Erfindung kann bei einer Schleusenanordnung nach Anspruch 11 oder bei einer vorangehenden Ausgestaltung vorgesehen sein, dass der Eingangsstation (3), welche von einem Gehäuse (39) umgeben ist, das einen zu einer angrenzenden Bereitstellungsstation (2) führenden Zugang (37) aufweist, wobei:
a) die Eingangsstation (3) mit einer gereinigten, gleichgerichteten Verdrängungsströmung (LF) betreibbar ist, die teilweise durch den Zugang (37) austritt;
b) die Bereitstellungsstation (2) zur Anlieferung der Gebinde (9;91,92,93) bestimmt ist, welche im Anlieferungszustand jeweils von einer Umhüllung (99) umgeben sind, um das Innenvolumen (90) des Gebindes (9;91,92,93) mit dessen Inhalt aseptisch zu erhalten; und
c) an der Bereitstellungsstation (2) eine an der Umhüllung (99) angebrachte Öffnung, die bis anhin mittels eines angesetzten Werkzeugs geschlossen bleibt, nun auf der der aus dem Zugang (37) austretenden Verdrängungsströmung (LF) zugewandten Seite durch Lösen des Werkzeugs freikommt, so dass das einzelne Gebinde (9;91,92,93) in die Eingangsstation (3) einbringbar ist; oder
d) an der Bereitstellungsstation (2) die Umhüllung (99) auf der der aus dem Zugang (37) austretenden Verdrängungsströmung (LF) zugewandten Seite mittels eines Werkzeugs auftrennbar ist, so dass das einzelne Gebinde (9;91,92,93) in die Eingangsstation (3) einbringbar ist.

Bei einer achten Ausgestaltung der Erfindung kann bei einer Schleusenanordnung nach Anspruch 11 oder bei einer vorangehenden Ausgestaltung vorgesehen sein, dass
a) zur Positionierung des in der zuerst noch geschlossenen Umhüllung (99) steckenden Gebindes (9;91,92,93) eine Auflage (20) vorgesehen ist;
b) zum Ausschieben des Gebindes (9;91,92,93) aus der offenen Umhüllung (99), bei auf das Gebinde (9;91,92,93) fliessender Verdrängungsströmung (LF), ein Vorschubmittel (22) dient; und
c) zum Zurückhalten der Umhüllung (99) ein Greifer (21), vorzugsweise als Sauger ausgebildet, dient.

Bei einer neunten Ausgestaltung der Erfindung kann bei einer Schleusenanordnung nach Anspruch 11 oder bei einer vorangehenden Ausgestaltung vorgesehen sein, dass
a) zumindest eine Fördereinrichtung (34, 35) zur Zuführung der in die Eingangsstation (3) eingebrachten Gebinde (9;91,92,93) an die Passagen (47,48) vorgesehen ist; und
b) die zumindest eine Fördereinrichtung (34,35) auf dem Prinzip der Magnetschwebetechnik beruht.

Bei einer zehnten Ausgestaltung der Erfindung kann bei einer Schleusenanordnung nach Anspruch 11 oder bei einer vorangehenden Ausgestaltung vorgesehen sein, dass
a) zum Transport der Gebinde (9;91,92,93) innerhalb der Eingangsstation (3) eine erste Fördereinrichtung (34) dient; während
b) eine zweite Fördereinrichtung (35) zum Heranschwenken der Gebinde (9;91,92,93) mit an der jeweiligen Passage (47, 48) angepressten Abdeckung (98) bestimmt ist; und
c) die zweite Fördereinrichtung (35) auch zum Entfernen bearbeiteter Gebinde (9;91,92,93) von der jeweiligen Passage (47,48) nutzbar ist.

Bei einer elften Ausgestaltung der Erfindung kann bei der zehnten Ausgestaltung vorgesehen sein, dass zur Positionierung der Gebinde (9;91,92,93) an der jeweiligen Passage (47,48) die zweite Fördereinrichtung (35) ein kardanisches Lager (351) umfasst.

Bei einer zwölften Ausgestaltung der Erfindung kann bei einer Schleusenanordnung nach Anspruch 11 oder bei einer vorangehenden Ausgestaltung vorgesehen sein, dass die zweite Fördereinrichtung (35) besteht aus:
a) einer Achse (350), durch welche sich eine erste Drehachse (D1) erstreckt;
b) zwei nebeneinander an der Achse (350) fixierten Gabeln (352), die jeweils um eine zweite Drehachse (D2) schwenkbar sind, welche sich rechtwinklig zur ersten Drehachse (D1) erstrecken; und
c) jeweils einen in der zugehörigen Gabel (352) aufgehängten und um eine dritte Drehachse (D3) schwenkbaren Träger (353), wobei die erste Drehachse (D1) und die dritte Drehachse (D3) zueinander parallel verlaufen und die Träger (353) zum Unterfassen der Ränder (941) der Behältnisse (94) beim An- bzw. Abschwenken von den Passagen (47,48) konfiguriert sind; wobei
d) zwei nebeneinander liegende kardanische Lager (351) jeweils gebildet sind aus einen U-förmigen Träger (353), der in der dritten Drehachse (D3) jeweils mit einer Gabel (352) verbunden ist, die in der zweiten Drehachse (D2) aufgehängt ist.

Bei einer dreizehnten Ausgestaltung der Erfindung kann bei einer Schleusenanordnung nach Anspruch 11 oder bei einer vorangehenden Ausgestaltung vorgesehen sein, dass in jeder Passage (47,48) eine Dichtung (470,480) sitzt, welche zusammen mit der Abdeckung (98) eines temporär angepressten Gebindes (9;91,92,93) und/oder mit einem temporär angepressten Deckel (51) an den Passagen (47,48), im Übergang von der Eingangsstation (3) zur Prozesskammer (43), Abdichtungen schafft.

Bei einer vierzehnten Ausgestaltung der Erfindung kann bei einer Schleusenanordnung nach Anspruch 11 oder bei einer vorangehenden Ausgestaltung vorgesehen sein, dass ein schwenkbarer Ausleger (50) einer in der Prozesskammer (43) positionierten Apparatur (5) den Deckel (51) trägt.

Bei einer fünfzehnten Ausgestaltung der Erfindung kann bei einer Schleusenanordnung nach Anspruch 11 oder bei einer vorangehenden Ausgestaltung vorgesehen sein, dass der zumindest eine Manipulator (6) oder auch der zusätzliche Manipulator (6) als Roboter ausgebildet sind.

Bei einer sechzehnten Ausgestaltung der Erfindung kann bei einer Schleusenanordnung nach Anspruch 11 oder bei einer vorangehenden Ausgestaltung vorgesehen sein, dass die Behältnisse (94) der Gebinde (9;91,92,93) die Gestalt wannenförmiger Tubs haben und die Artikel (960), **z.B.** Phiolen, Vials oder Spritzen sind, die in systematisch angeordneten Aufnahmekonturen von Nestern (96) stecken.

### Kurzbeschreibung der beigefügten Zeichnungen

Es zeigen:
Figur 1A - ein mit dem erfindungsgemässen Verfahren in der zugehörigen Schleusenanordnung zu verarbeitendes Gebinde mit geschlossener Umhüllung;
Figur 1B - das Gebinde gemäss Figur 1A ohne die Umhüllung in Explosivansicht;

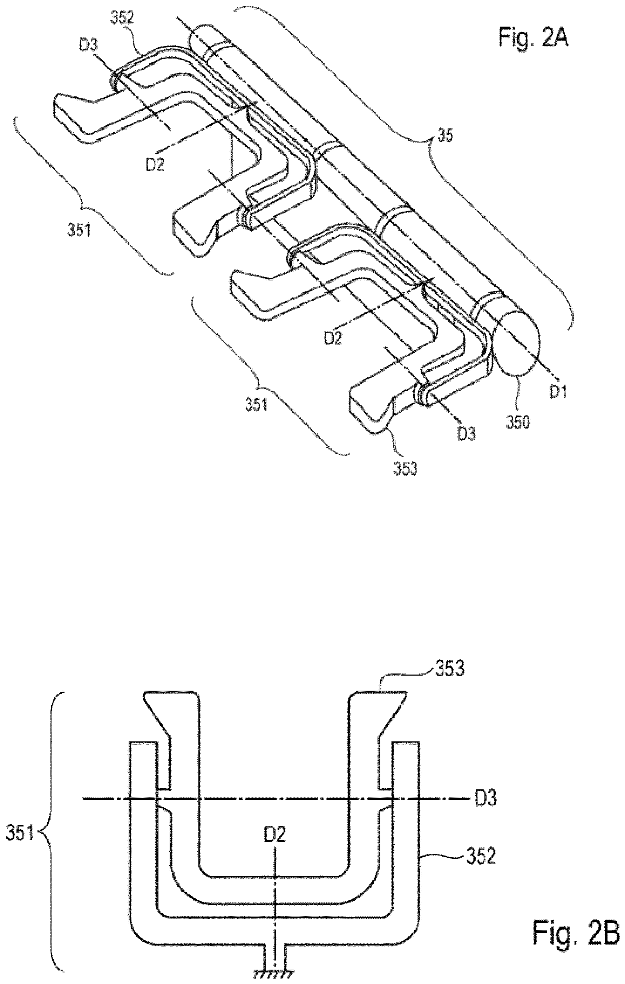

Figur 2A - die zweite Fördereinrichtung als Bestandteil der Schleusenanordnung, bestehend aus der Achse und daran fixierten zwei kardanischen Lagern, in Perspektivansicht;
Figur 2B - ein kardanisches Lager aus Figur 2A, in Draufsicht;
Figuren 3A bis 15D: die Gesamtheit eines Verfahrensdurchlaufs mit jeweils vier Figuren der Schleusenanordnung, nämlich deren vordere Sektion gemäss Figuren A, hintere Sektion gemäss Figuren B, in Draufsicht gemäss Figuren C und als prinzipielle Seitenansicht gemäss Figuren D;

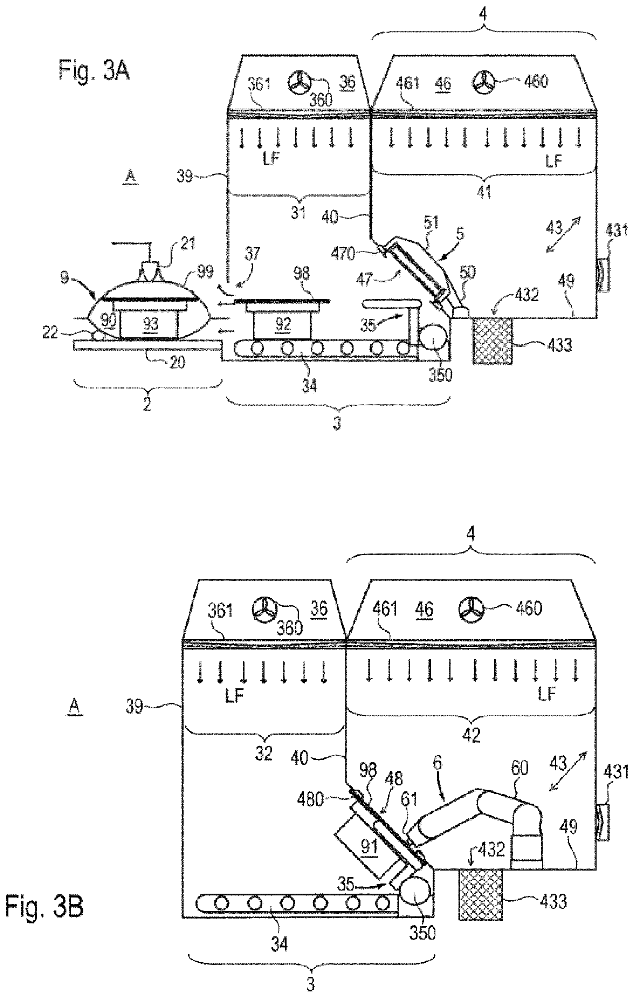

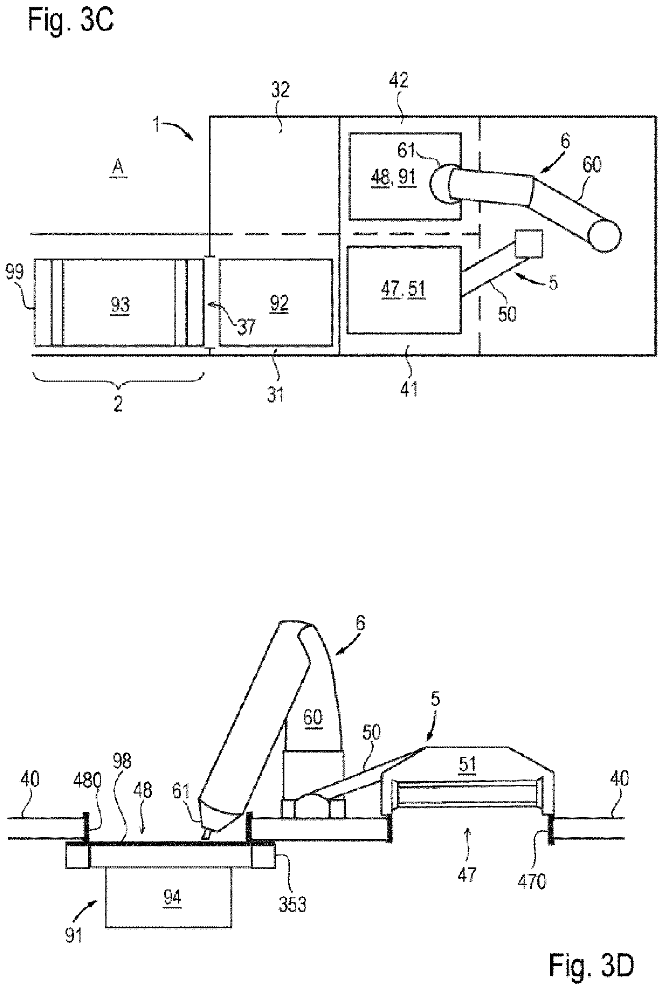

Figuren 3A bis 3D - Prozessphase 1 (Startposition)

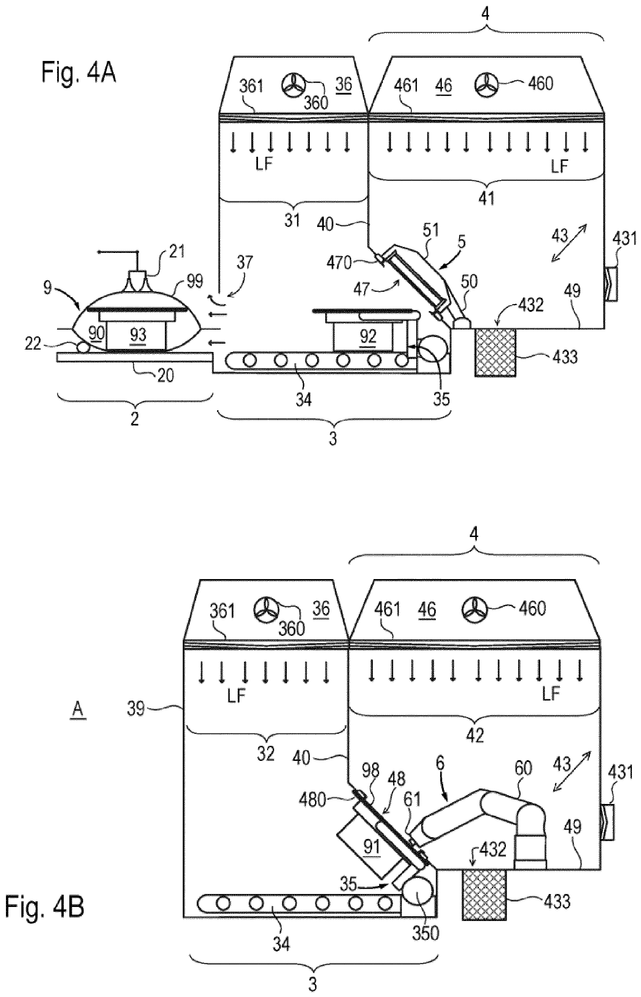

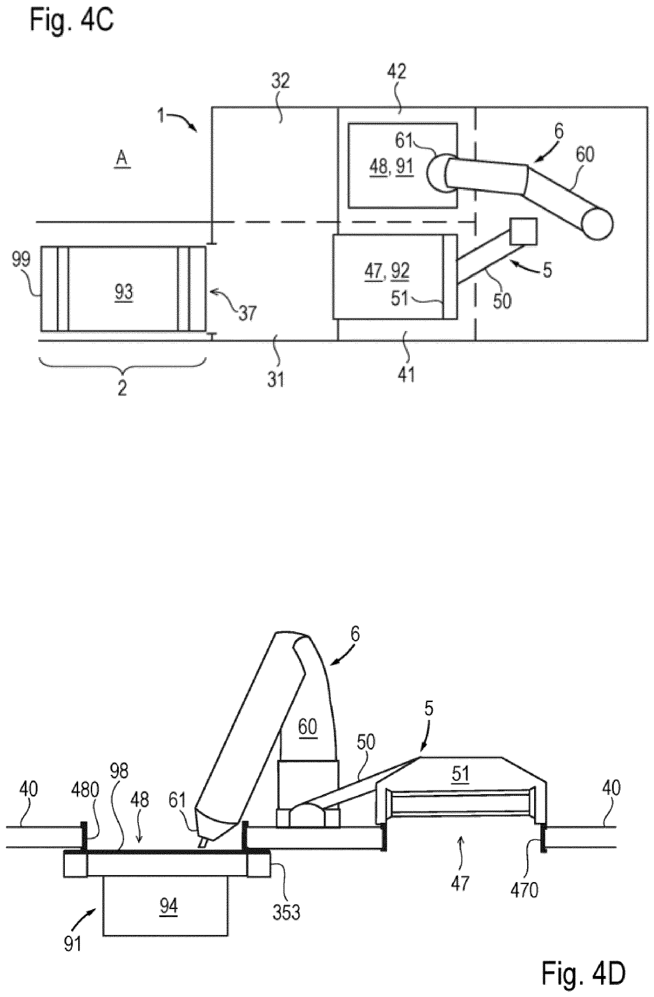

Figuren 4A bis 4D - Prozessphase 2

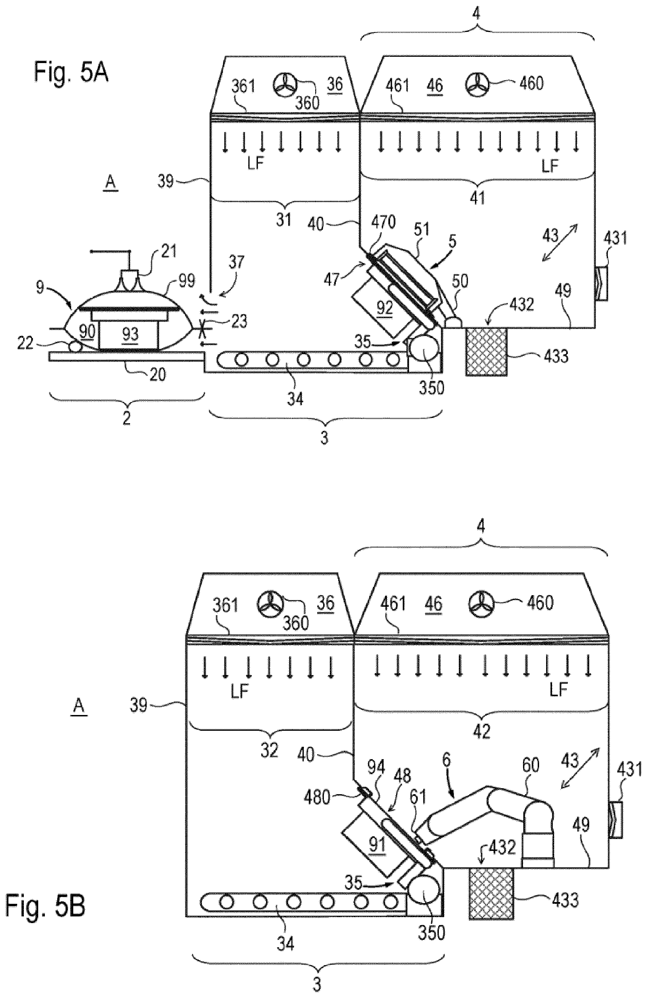

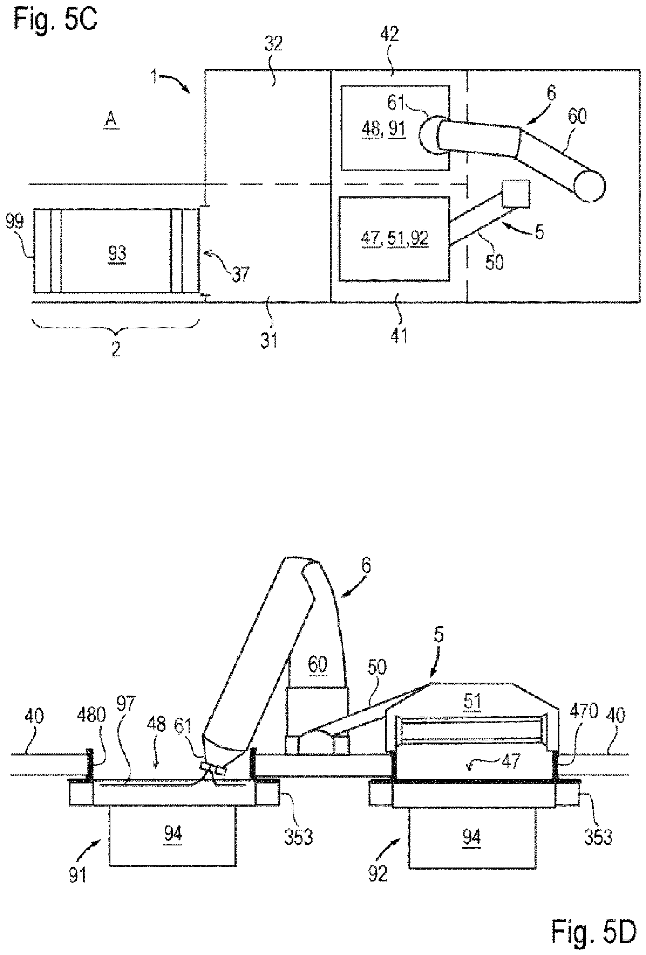

Figuren 5A bis 5D - Prozessphase 3

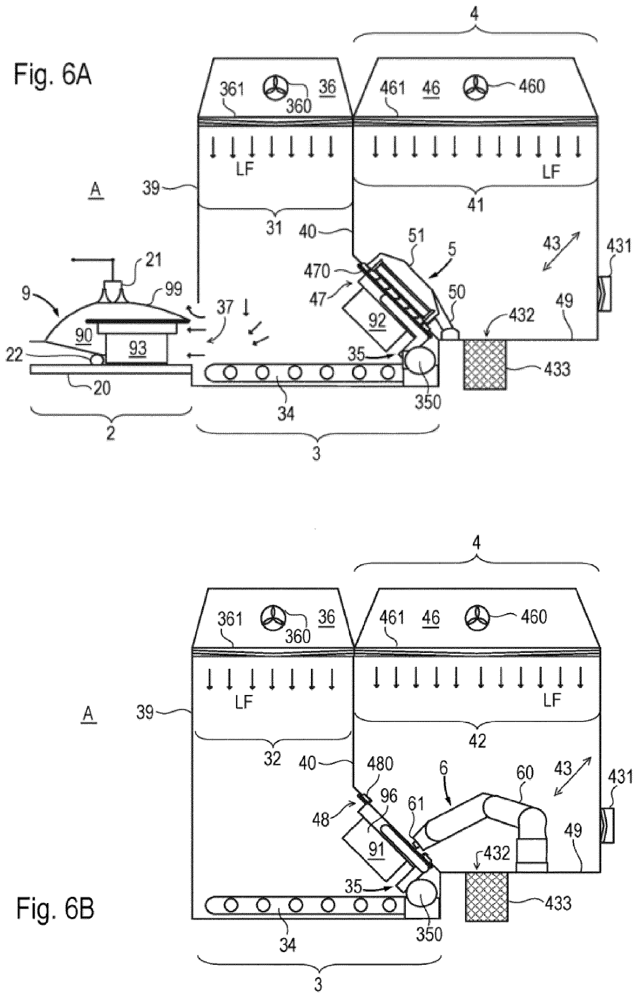

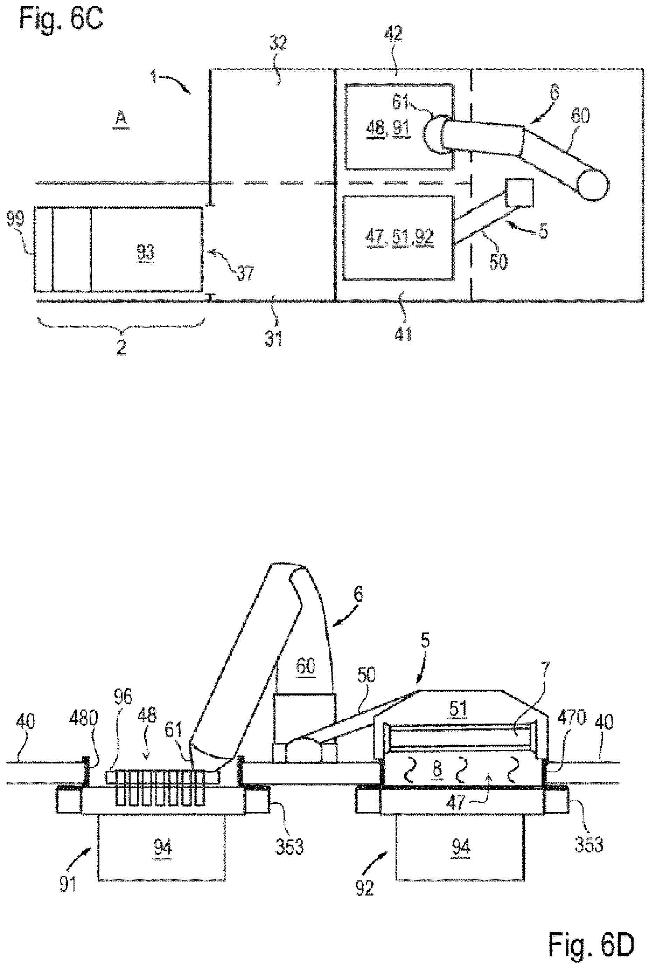

Figuren 6A bis 6D - Prozessphase 4

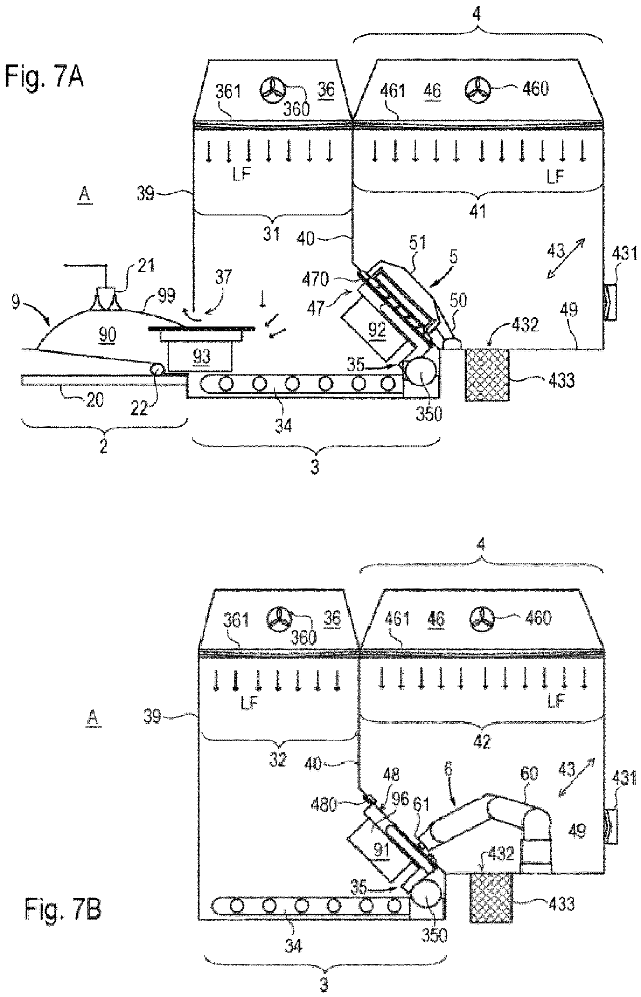

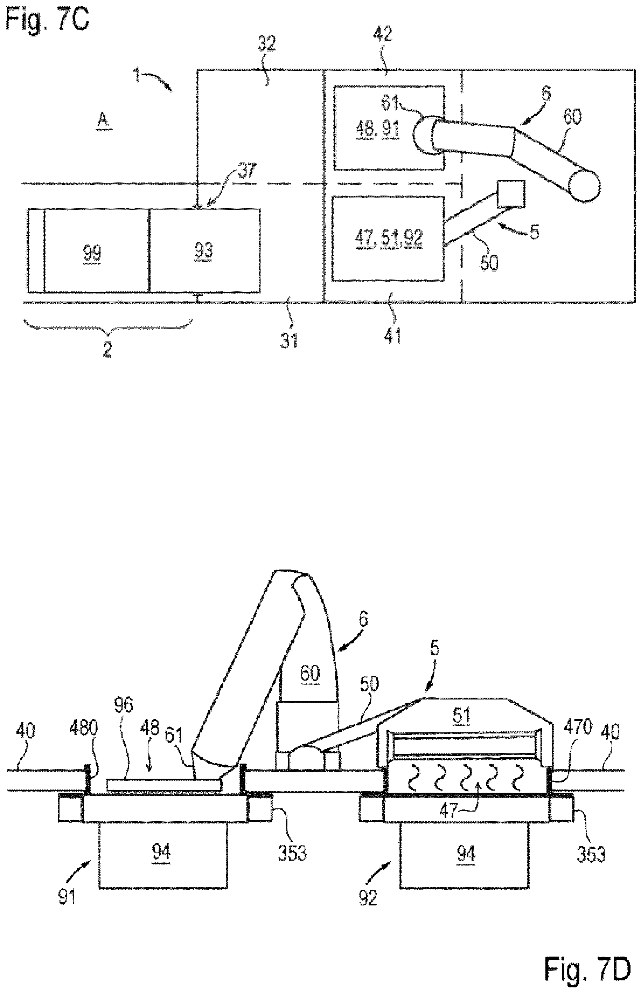

Figuren 7A bis 7D - Prozessphase 5

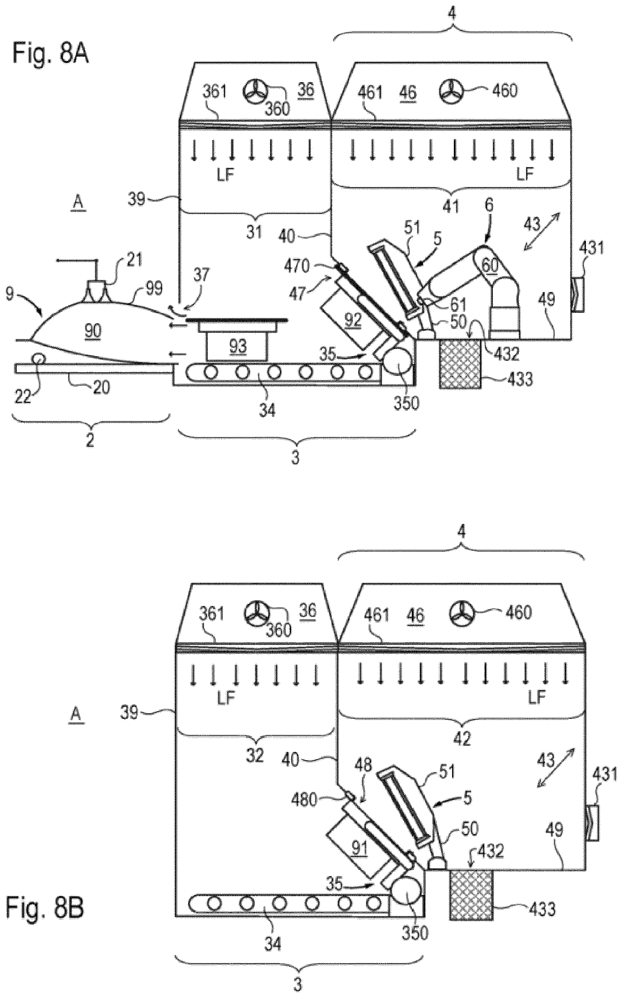

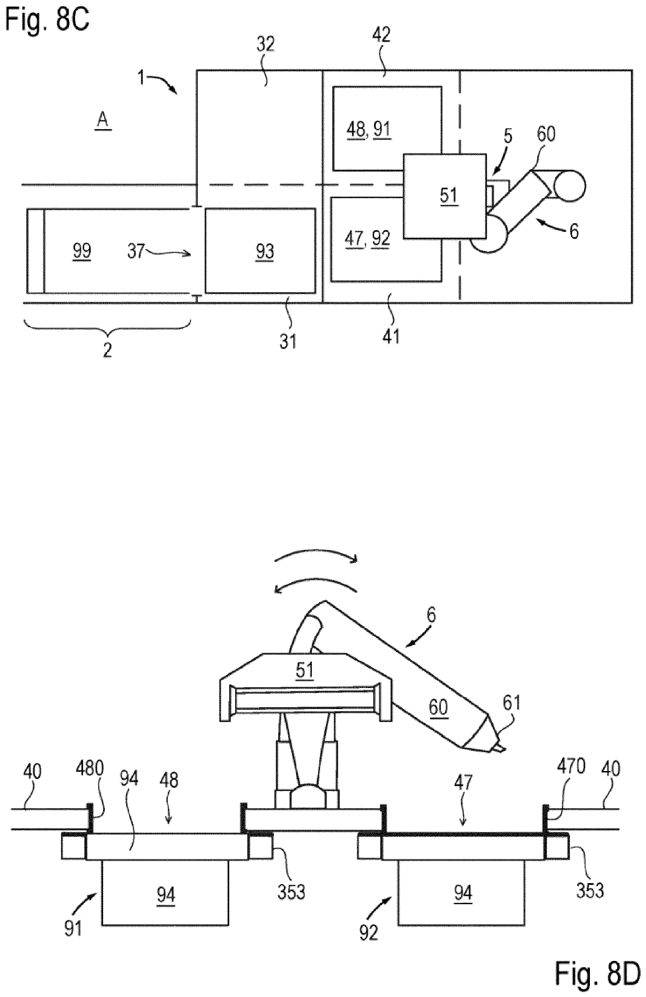

Figuren 8A bis 8D - Prozessphase 6

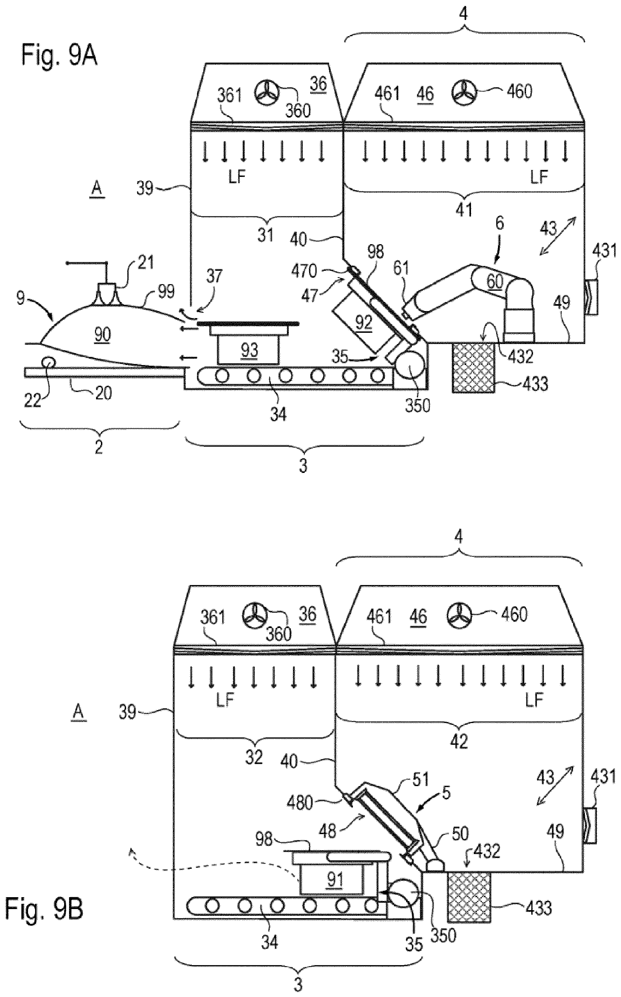

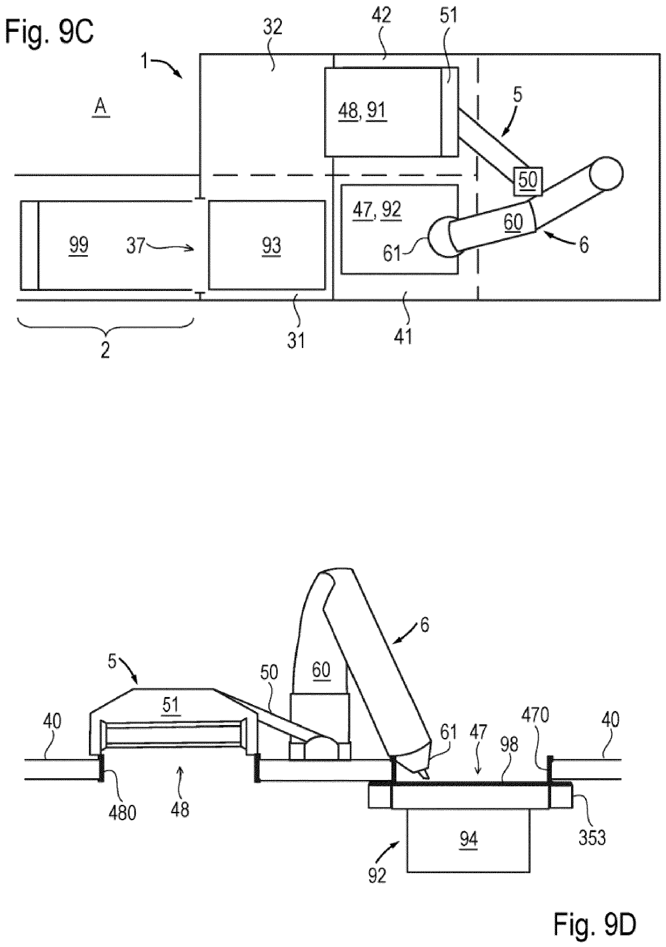

Figuren 9A bis 9D - Prozessphase 7

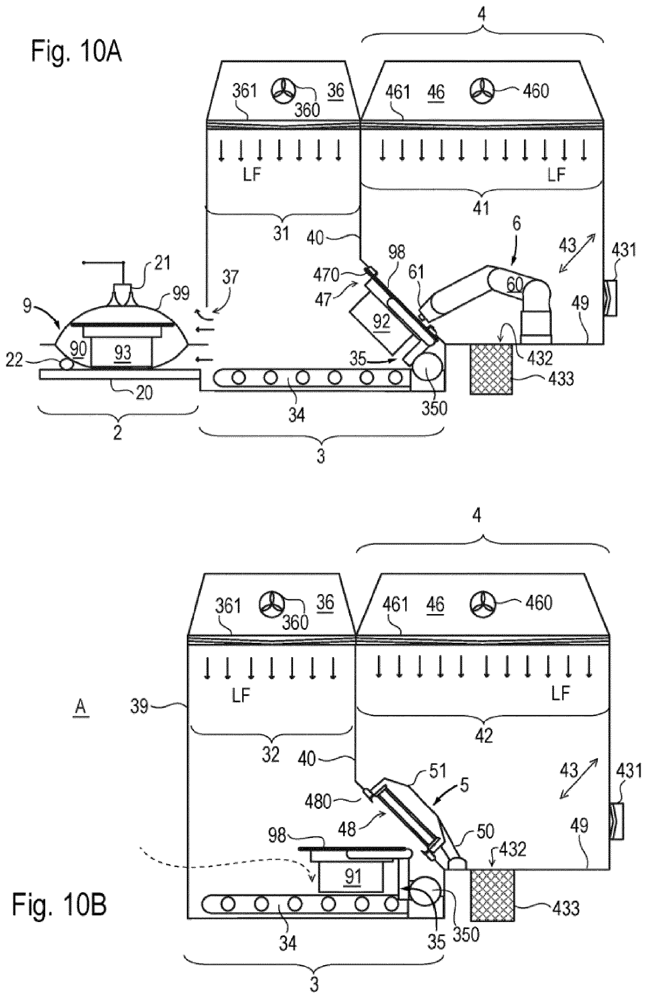

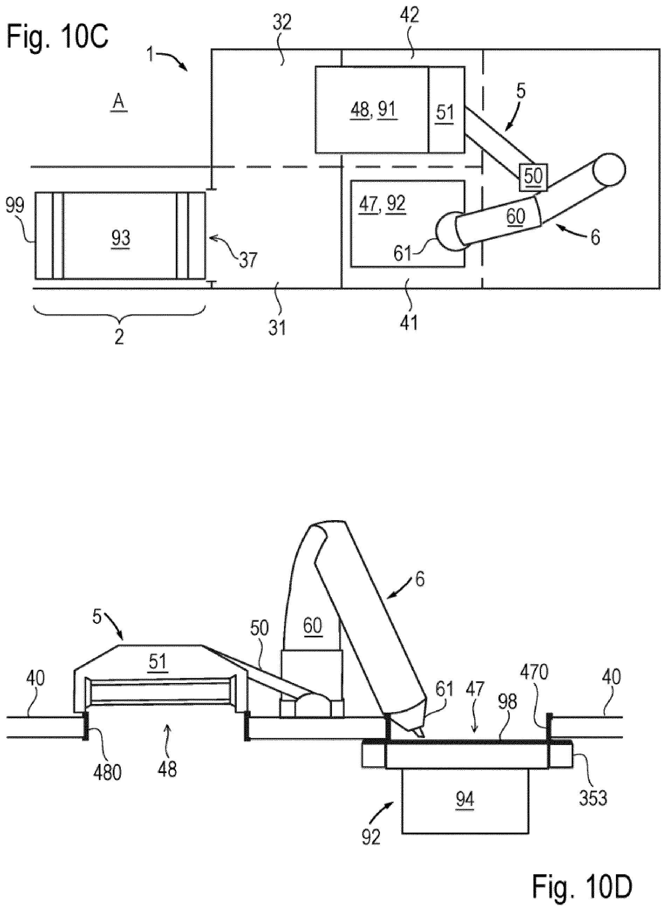

Figuren 10A bis 10D - Prozessphase 8

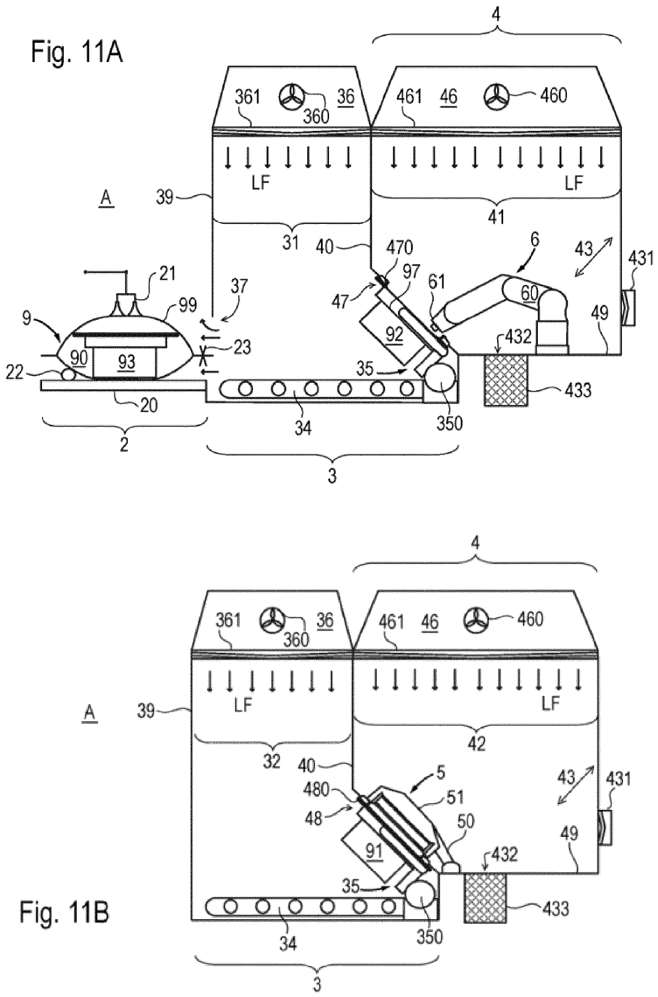

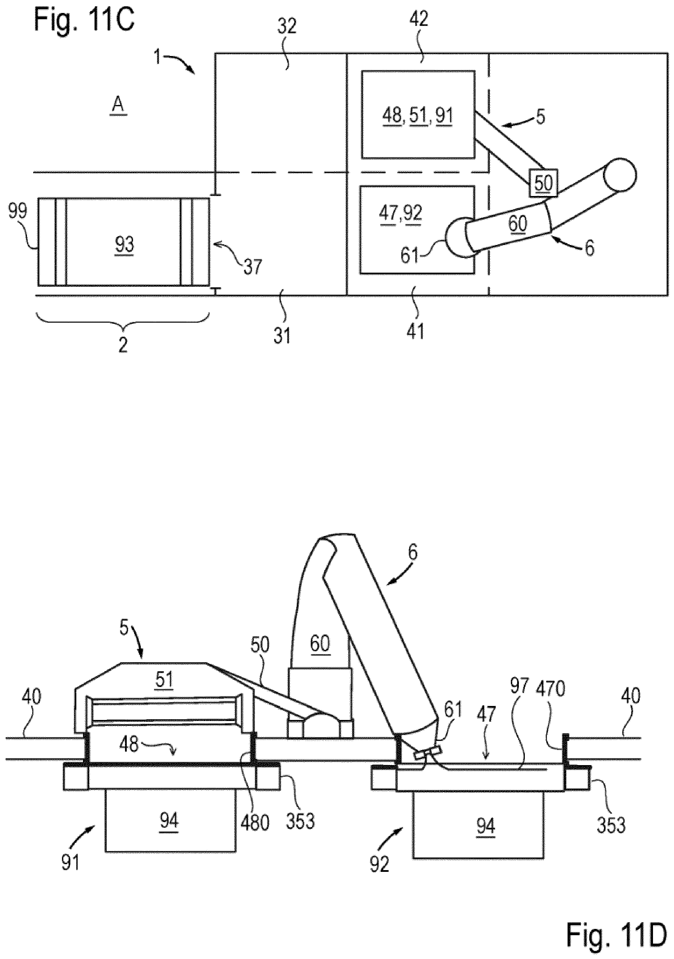

Figuren 11A bis 11D - Prozessphase 9

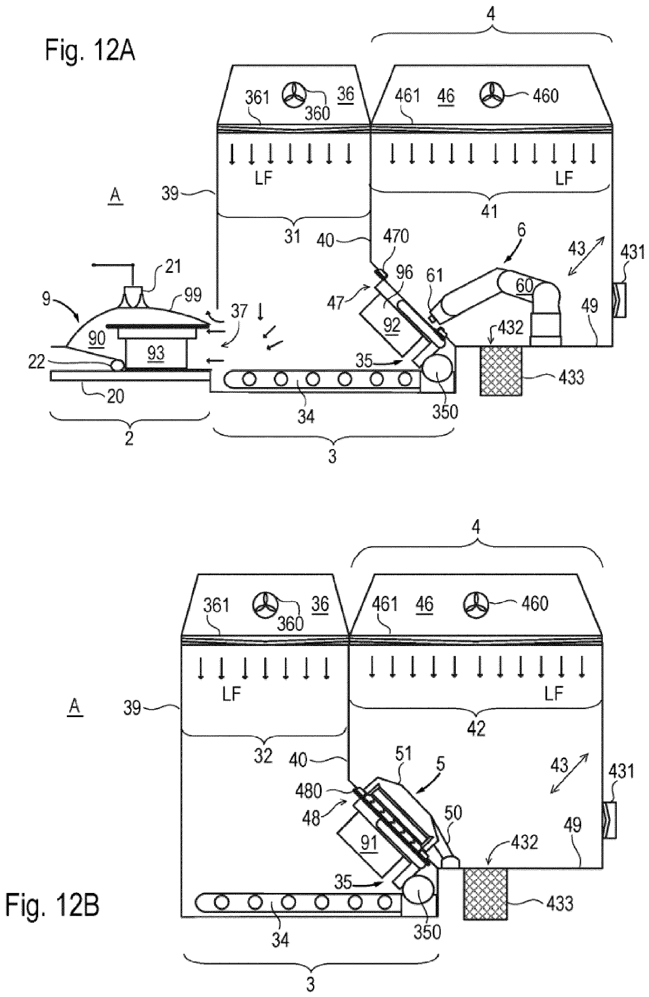

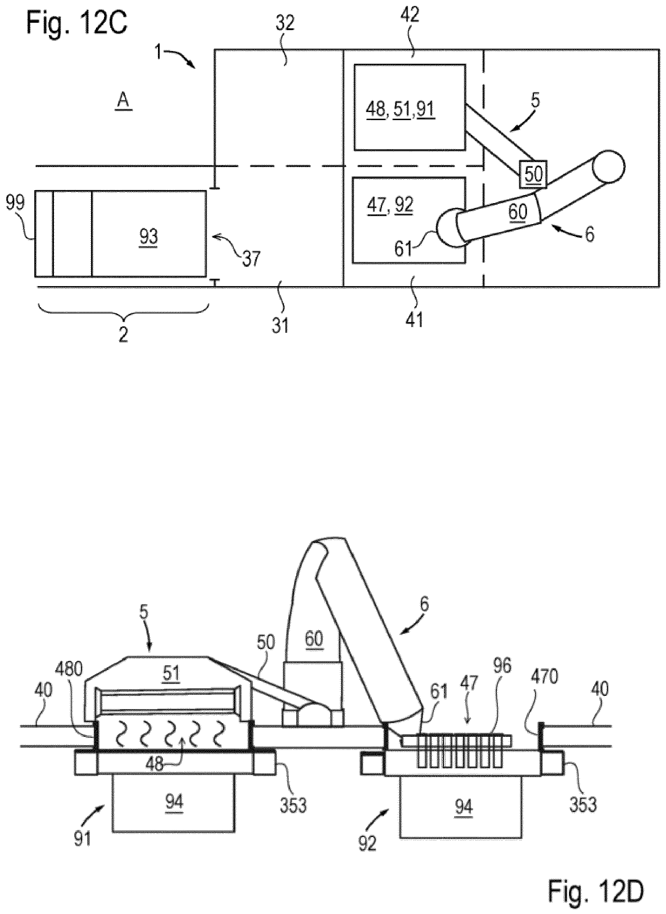

Figuren 12A bis 12D - Prozessphase 10

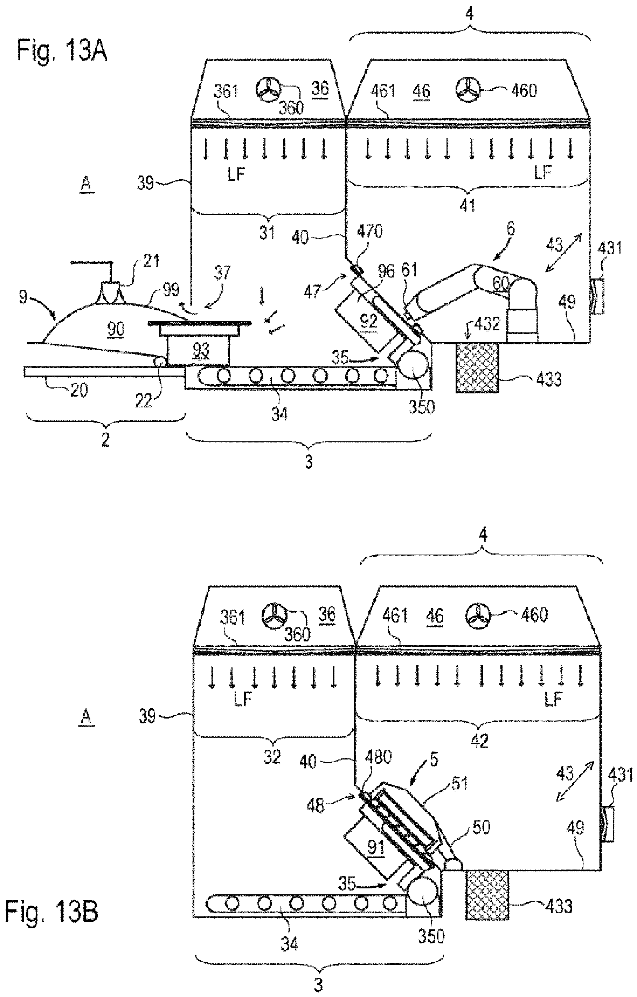

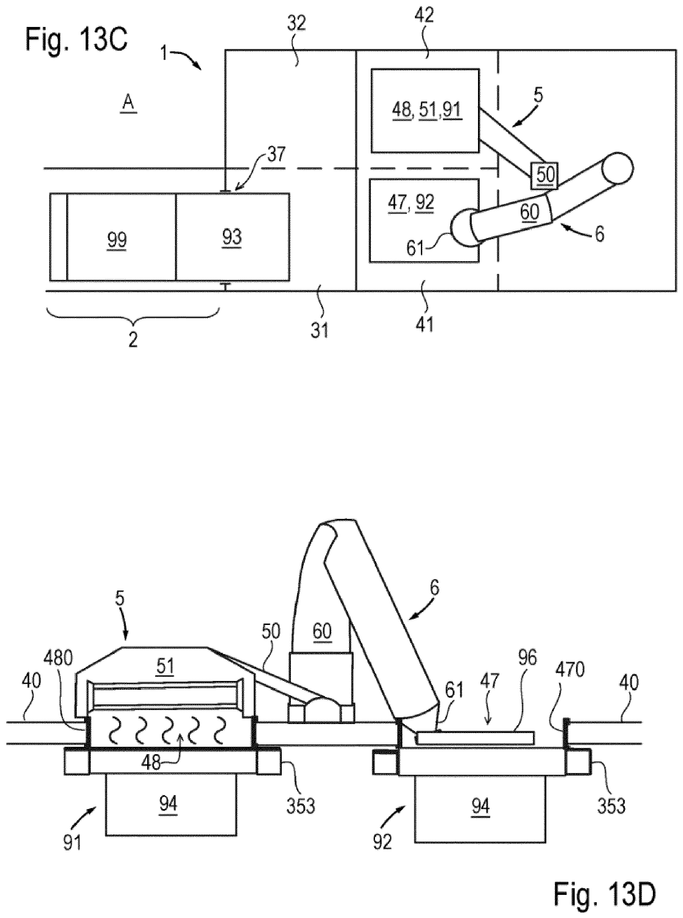

Figuren 13A bis 13D - Prozessphase 11

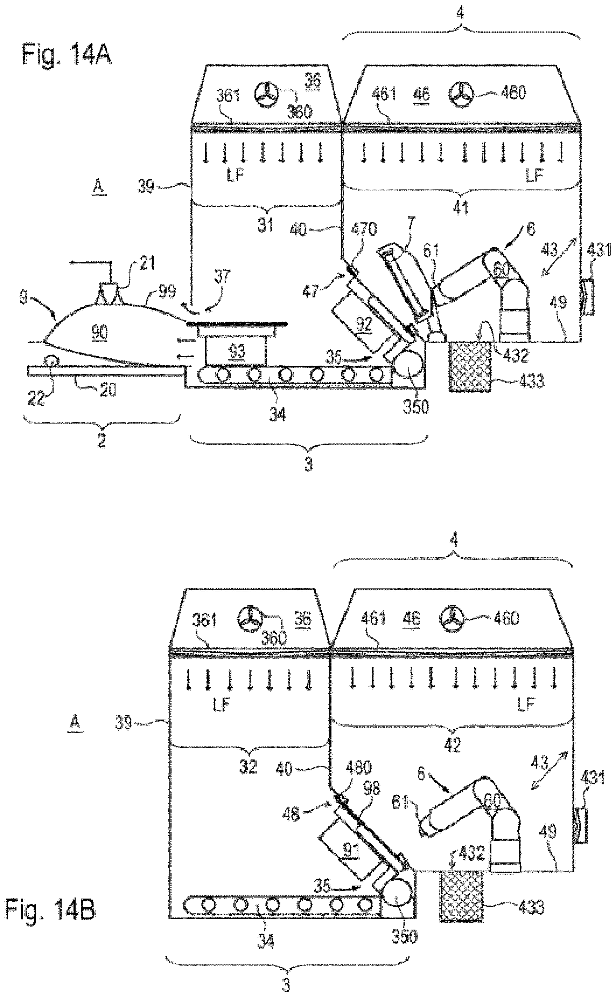

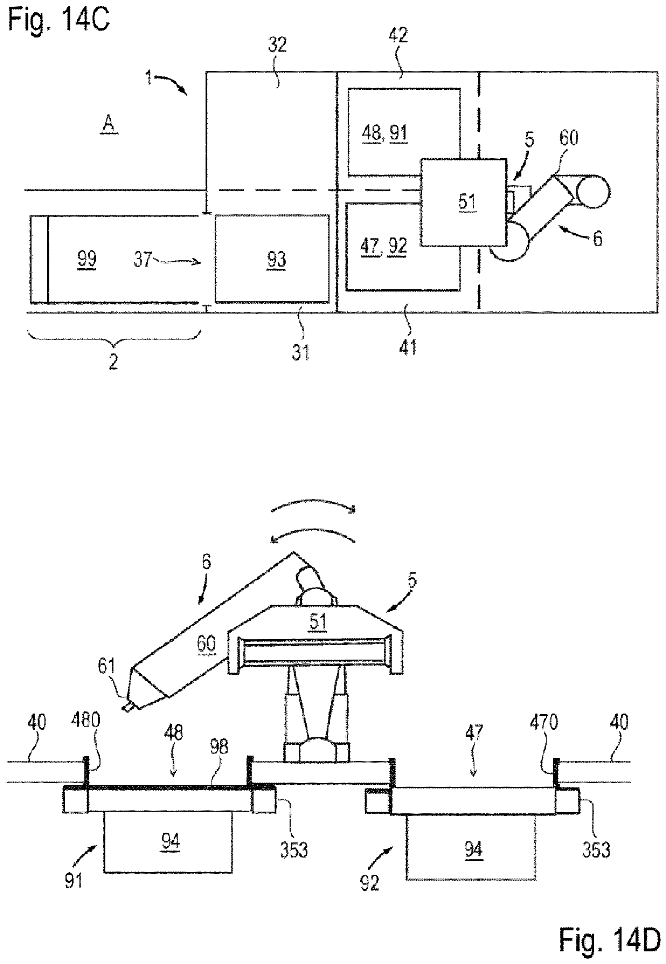

Figuren 14A bis 14D - Prozessphase 12

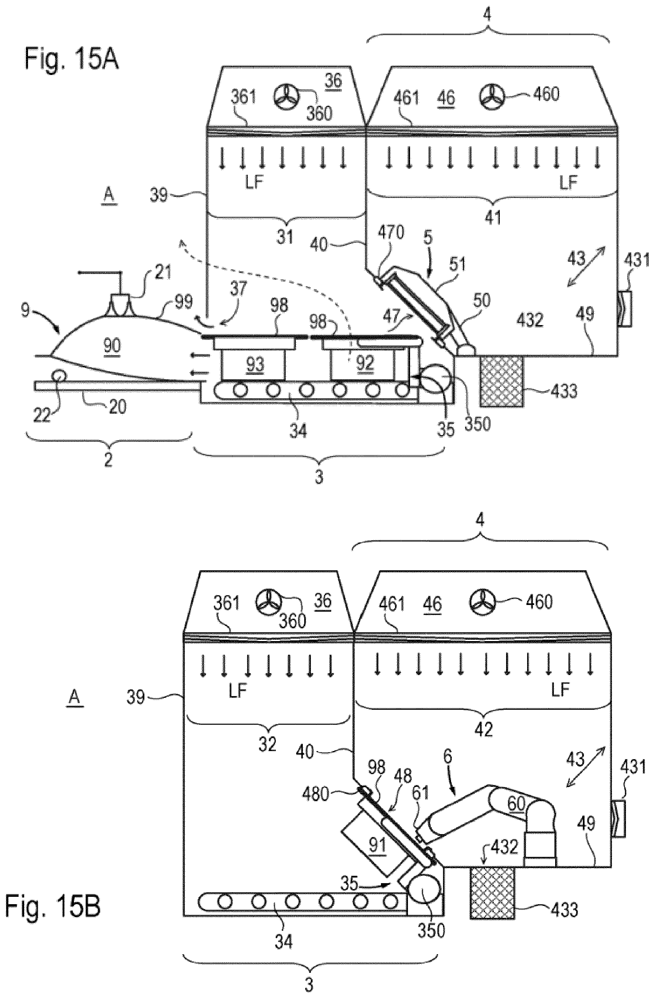

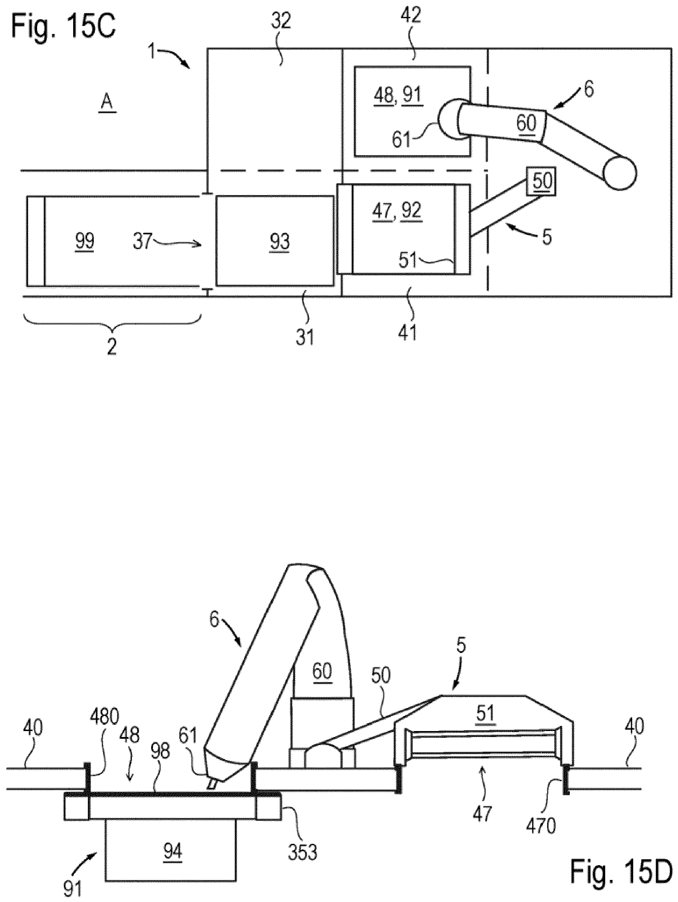

Figuren 15A bis 15D - Prozessphase 13

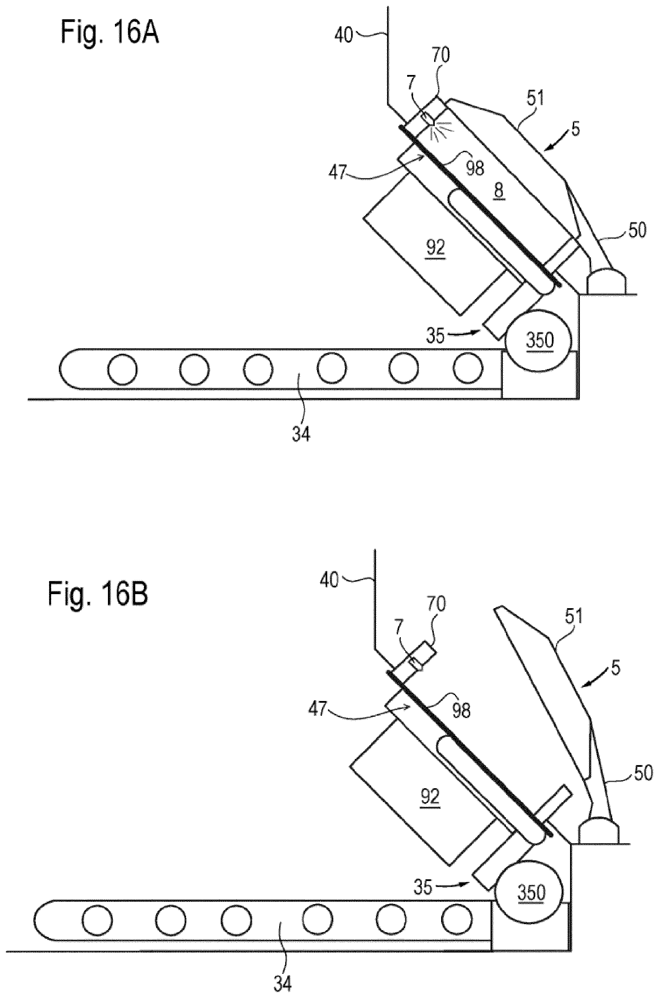

Figur 16A - eine alternative Anordnung mit der Dekantaminationsvorrichtung ausserhalb des Deckels, in einer Halterung sitzend, Gebinde an der Passage mittels der hochgeschwenkten zweiten Fördereinrichtung angedrückt, Deckel geschlossen, Dekantaminationsvorrichtung aktiviert; und
Figur 16B - die Anordnung gemäss Figur 16A mit geöffnetem Deckel und inaktiver Dekantaminationsvorrichtung.

### Ausführungsbeispiel

Mit Bezug auf die beiliegenden Zeichnungen erfolgt nachstehend die detaillierte Beschreibung des erfindungsgemässen Verfahrens und der erfindungsgemässen Schleusenanordnung zum Einschleusen von Artikeln aus einem Gebinde in die Prozesskammer eines Containments unter aseptischen Bedingungen. Wie die beigefügten Zeichnungen, betrifft die nachfolgend beschriebene Ausführungsform den Transfer von Artikeln aus Gebinden in die Prozesskammer durch zwei von der Eingangsstation in die Prozesskammer führende Passagen. Jede der Passagen lässt sich abwechselnd von einem in der Prozesskammer herangeführten Deckel verschliessen, in welchem das Emissionselement einer Dekontaminationsvorrichtung integriert sein kann.

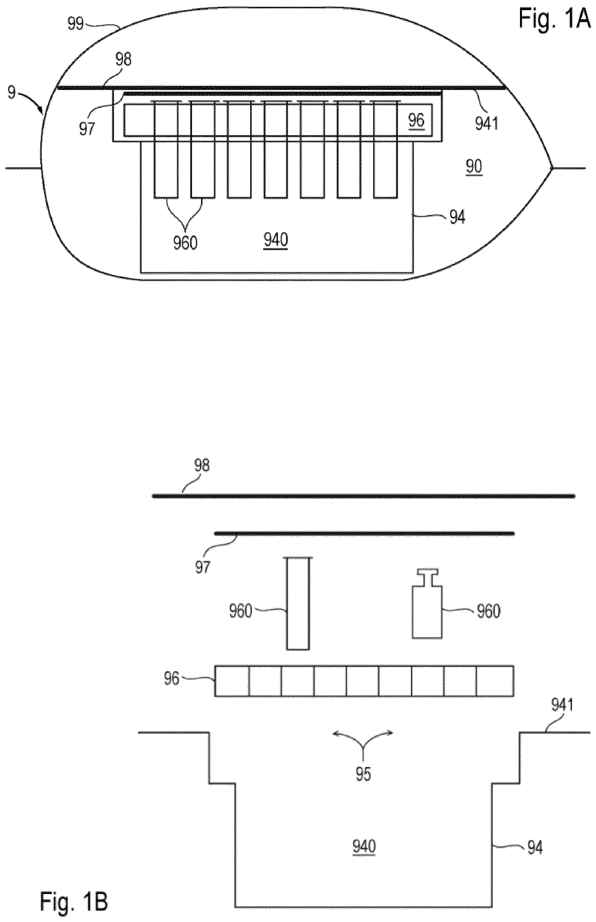

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen. Figuren 1A und 1B

Ein einzelnes Gebinde **9** umfasst:
- ein Behältnis **94** mit einem aseptischen Innenraum **940** und einem Durchlass **95,** der mit einer Abdeckung **98** verschlossen ist;
- ein im Innenraum **940** gelagertes Nest **96,** in dessen muldenförmigen Aufnahmekonturen die Artikel **960** stecken, welche nach Öffnen der Abdeckung **98** in der Prozesskammer **43** des Containments **4** zu behandeln sind;
- optional eine zwischen der Abdeckung **98** und dem Nest **96,** über den Artikeln **960** eingefügte Zwischenlage **97;** sowie
- eine im Anlieferungszustand das gesamte Gebinde **9** umgebende, geschlossene Umhüllung **99,** die das Innenvolumen **90** steril hält.

Das Behältnis **94** ist z.B. ein wannenförmiges Tub. Die Artikel **960** sind insbesondere Phiolen, Vials oder Spritzen. Von der Abdeckung **98** und der Umhüllung **99** bestehen vorzugsweise zumindest Flächenanteile aus einem semipermeablen Vliesgewebe, wie Tyvek^{®}. Die Abdeckung **98** ist typischerweise auf dem den Durchlass **95** des Behältnisses **94** umlaufenden Rand **941** versiegelt und bewahrt so den Innenraum **940** des Behältnisses **94** in sterilem Zustand.

### Figuren 2A und 2B

Als Bestandteil der Schleusenanordnung **1** sind die frontseitige erste Zone **31** und die rückseitige zweite Zone **32** der Eingangsstation **3** jeweils mit einer zweiten Fördereinrichtung **35** ausgestattet. Eine solche Fördereinrichtung **35** besteht aus einer Achse **350** und daran fixierten zwei kardanischen Lagern **351.** Durch die Achse **350** erstreckt sich eine erste Drehachse **D1.** Zwei nebeneinander an der Achse **350** fixierte Gabeln **352** sind jeweils um eine zweite Drehachse **D2** schwenkbar, welche sich rechtwinklig zur ersten Drehachse **D1** erstrecken. In jeder Gabel **352** ist ein um eine dritte Drehachse **D3** schwenkbarer Träger **353** aufgehängt, wobei die erste Drehachse **D1** und die dritte Drehachse **D3** zueinander parallel verlaufen. Die Träger **353** sind zum Unterfassen der Ränder **941** der Behältnisse **94** beim An- bzw. Abschwenken von den von der Eingangsstation **3** in die Prozesskammer **43** führenden Passagen **47,48** konfiguriert. Figuren 3A bis 3D Aufbau der Schleusenanordnung **1**

Die im Aufstellraum **A** installierte Schleusenanordnung **1** strukturiert sich in eine Eingangsstation **3,** eine dieser vorgelagerte Bereitstellungsstation **2** und das sich an die Eingangsstation **3** anschliessende Containment **4** mit dessen Prozesskammer **43.** Die Bereitstellungsstation **2** besitzt die Auflage **20,** um darauf neue, dritte Gebinde **93** mit zunächst noch mit verschlossener Umhüllung **99** zu positionieren. Das Vorschubmittel **22** dient dem Ausschieben solcher dritten Gebinde **93** aus der geöffneten Umhüllung **99** von der Auflage **20** durch den Zugang **37** im die Eingangsstation **3** umgebenden Gehäuse **39** auf die erste Fördereinrichtung **34** in der frontseitigen ersten Zone **31** der Eingangsstation **3.** Während des Ausschiebens hält der in der Bereitstellungsstation **2** vorhandene Greifer **21** die Umhüllung **99** zurück. An die erste Fördereinrichtung **34** schliesst sich die zweite Fördereinrichtung **35** an. Im Plenum **36** der frontseitigen ersten Zone **31** der Eingangsstation **3** sind ein Zuluftventilator **360** sowie Zuluftfilter **361** zur Erzeugung eines gereinigten, abwärts laminar strömenden Luftstromes **LF** angeordnet.

Zwischen der frontseitigen ersten Zone **31** der Eingangsstation **3** und der frontseitigen ersten Zone **41** der Prozesskammer **43** erstreckt sich die Trennwand **40,** welche die erste Passage **47** zwischen den Zonen **31,41** aufweist. In der ersten Passage **47** sitzt die Dichtung **470.** Auch im Plenum **46** der frontseitigen ersten Zone **41** des Containments **4** sitzen ein Zuluftventilator **460** und ein Zuluftfilter **461** zur Erzeugung eines gereinigten, abwärts laminar strömenden Luftstromes **LF.** Im die Prozesskammer **43** umgebenden Gehäuse **49** sind seitlich und/oder alternativ in dessen Boden ein Abluftfilter **431** und im Boden eine Abfallöffnung **432** vorgesehen, die zu einem Depot **433** führt.

In der sich über die frontseitige erste Zone **41** und die rückseitige zweite Zone **42** erstreckende gemeinsame Prozesskammer **43** ist eine Apparatur **5** mit dem Ausleger **50** und dem daran gehaltenen Deckel **51** stationiert, in dem die Dekontaminationsvorrichtung **7** integriert ist. Durch Schwenken des Auslegers **50** kann der Deckel **51** über Zwischenstellungen an die erste Passage **47** oder die zweite Passage **48** herangeführt werden.

In der Eingangsstation **3** liegt hinter der ersten Zone **31** die rückseitige zweite Zone **32,** und im Containment **4** liegt hinter der ersten Zone **41** die rückseitige zweite Zone **42.** Äquivalent zur ersten Zone **31** ist die zweite Zone **32** ebenfalls in deren Plenum **36** mit einem Zuluftventilator **360** und einem Zuluftfilter **361** zur Erzeugung eines Luftstromes **LF** ausgestattet. Wiederum vorhanden sind eine erste Fördereinrichtung **34** und die sich daran anschliessende zweite Fördereinrichtung **35** an. Gleichfalls äquivalent zur ersten Zone **41** hat die zweite Zone **42** das Plenum **46** mit Zuluftventilator **460** und Zuluftfilter **461** zur Erzeugung des Luftstromes **LF.**

Bei kleineren Anlagen können ein gemeinsamer Zuluftventilator **360** und ein gemeinsamer Zuluftfilter **361** für die erste Zone **31** und die zweite Zone **32** sowie ein gemeinsamer Zuluftventilator **460** und ein gemeinsamer Zuluftfilter **461** für die erste Zone **41** und die zweite Zone **42** genügen. Bei grösseren Anlagen wird man für die Zonen **31,32** bzw. **41,42** mehrere Zuluftventilatoren **360,460** bzw. Zuluftfilter **361,461** vorsehen. In der rückseitigen zweiten Zone **42** sind ebenfalls im Gehäuse **49** seitlich und/oder alternativ in dessen Boden der Abluftfilter **431** und im Boden die zu einem Depot **433** führende Abfallöffnung **432** vorhanden.

Zwischen der rückseitigen zweiten Zone **32** der Eingangsstation **3** und der rückseitigen zweiten Zone **42** der Prozesskammer **43** erstreckt sich erneut die Trennwand **40,** welche nun die zweite Passage **48** zwischen den Zonen **32,42** aufweist. In dieser zweiten Passage **48** sitzt die Dichtung **480.** In der für die erste und zweite Zone **41,42** gemeinsamen Prozesskammer **43** ist ein Manipulator **6** mit dem Ausleger **60** und dem daran befindlichen Werkzeugkopf **61** stationiert. Durch Schwenken des Auslegers **60** kann der Werkzeugkopf **61** über Zwischenstellungen an die erste Passage **47** oder die zweite Passage **48** herangeführt werden.

### Ablauf in Prozessphase 1 (Startposition)

- In Bereitstellungsstation **2:**
   Ein neues, drittes Gebinde **93,** noch mit verschlossener Umhüllung **99,** ist auf der Auflage **20** angeliefert, befindet sich vor dem Zugang **37** und wird von durch diesen ausfliessender Strömung **LF** angeströmt.
- In Eingangsstation **3,** in deren frontseitiger erster Zone **31:**
   Ein zweites Gebinde **92,** bereits aus der Umhüllung **99** befreit, befindet sich auf der ersten Fördereinrichtung **34.** Vonseiten der ersten Zone **31** ist die erste Passage **47** quasi offen.
- In Eingangsstation **3,** rückseitige zweite Zone **32:**
   Die zweite Passage **48** in die Prozesskammer **43** des Containments **4** ist vom angedrückten ersten Gebinde **91** mit dessen Abdeckung **98** verschlossen, jedoch bereits dekontaminiert.
- Im Containment **4,** frontseitige erste Zone **41:**
   Die erste Passage **47** in die Prozesskammer **43** ist vom Deckel **51** mit der darin integrierten Dekontaminationsvorrichtung **7** verschlossen. Der Deckel **51** wurde von der Apparatur **5** mit dem geschwenkten Ausleger **50** aus der Prozesskammer **43** heraus herangeführt.
- Im Containment **4,** rückseitige zweite Zone **42:**
   Mit einem Werkzeug, welches am Werkzeugkopf **61** eines geschwenkten Auslegers **60** des in der Prozesskammer **43** stationierten Manipulators **6** montiert ist, wird die Abdeckung **98** geöffnet, z.B. aufgeschnitten, und entfernt.

Bei der nachstehenden Beschreibung des Ablaufs in den weiteren Prozessphasen 2 bis 13 ( Figuren 4A bis 15D ) werden zur Vermeidung von Wiederholungen jeweils nur die Veränderungen zur vorherigen Prozessphase aufgelistet.

### Ablauf in Prozessphase 2 (Figuren 4A bis 4D)

- In Eingangsstation **3,** frontseitige erste Zone **31:**
   Das zweite Gebinde **92,** ohne Umhüllung **99,** noch auf der ersten Fördereinrichtung **34** stehend, wird von der zweiten Fördereinrichtung **35** übernommen.

### Ablauf in Prozessphase 3 (Figuren 5A bis 5D)

- In Bereitstellungsstation **2:**
   Die bis anhin verschlossene Umhüllung **99** um das neue, dritte Gebinde **93** wird mittels des Werkzeugs **23** geöffnet, z.B. aufgeschnitten; hierbei hält der Greifer **21** die Umhüllung **99.** Alternativ kann ein zuvor an der Umhüllung **99** angebrachter Schnitt, den man zunächst, z.B. zuklemmt, jetzt freigegeben werden.
- In Eingangsstation **3,** frontseitige erste Zone **31:**
   Von der zweiten Fördereinrichtung **35** wurde das zweite Gebinde **92** an die zweite Passage **48** herangeführt, welche nun beidseitig vom zweiten angedrückten Gebinde **92** bzw. aufgesetzten Deckel **51** verschlossen ist.
- Im Containment **4,** rückseitige zweite Zone **42:**
   Mit einem Werkzeug, das am Werkzeugkopf **61** des schwenkbaren Auslegers **60** des Manipulators **6** installiert ist, wird bei offener Abdeckung **98** die optional vorhandene Zwischenlage **97** aus dem ersten Gebinde **91** entnommen.

### Ablauf in Prozessphase 4 (Figuren 6A bis 6D)

- In Bereitstellungsstation **2:**
   Das neue, dritte Gebinde **93** wird aus der aufseiten des Zugangs **37** offenen Umhüllung **99** vom bewegten Vorschubmittel **22** sukzessive in Richtung Zugang **37** ausgeschoben und dabei von der gereinigten Strömung **LF** bestrahlt. Dies verhindert das Umspülen des Gebindes **93** mit viel unreiner Umgebungsluft aus dem Aufstellraum **A.** Vom Greifer **21** wird die Umhüllung **99** festgehalten.
- Im Containment **4,** frontseitige erste Zone **41:**
   Die Dekontaminationsvorrichtung **7** wird aktiviert, um das zwischen Deckel **51,** Abdeckung **98** auf erstem Gebinde **91,** der die erste Passage **47** umrandende Dichtung **470** und möglichen freiliegenden Wandungsflächen im Umfeld **8** vorhandene Gasvolumen und alle davon umspülten Oberflächen zu dekontaminieren.
- Im Containment **4,** rückseitige zweite Zone **42:**
   Mit einem vom Manipulator **6** herangeführten Werkzeug werden aus dem Innenvolumen **90** aus dem Behältnis **94** des ersten Gebindes **91** das Nest **96,** zusammen mit den darin gelagerten Artikeln **960** oder nur die Artikel **960,** in die Prozesskammer **43** zur weiteren Verarbeitung transferiert.

### Ablauf in Prozessphase 5 (Figuren 7A bis 7D)

- In Bereitstellungsstation **2** und Eingangsstation **3,** frontseitige erste Zone **31:**
   Das neue, dritte Gebinde **93** wird durch pushende Bewegung des Vorschubmittels **22** aus der Umhüllung **99** heraus, durch den Zugang **37** im Gehäuse **39** und gegen die gereinigte Strömung **LF,** auf die erste Fördereinrichtung **34** in die erste Zone **31** der Eingangsstation 3 geschoben. Das Ausschieben des dritten Gebindes **93** aus der Umhüllung **99** geschieht direkt auf die erste Fördereinrichtung **34,** um eine Berührung zwischen Gebinde **93** und Auflage **20** zu vermeiden und so keine Übertragung von Verunreinigungen zu erlauben. Der Greifer **21** hält dabei die Umhüllung **99** zwecks Entsorgung zurück.
- Im Containment **4,** rückseitige zweite Zone **42:**
   In das in vorheriger Prozessphase geleerte Behältnis **94** wird ein aus früherem oder gegenwärtigem Produktionszyklus aus der Prozesskammer **43** stammendes Nest **96** mittels des Manipulators **6** in dieses Behältnis **94** rückgeführt.

### Ablauf in Prozessphase 6 (Figuren 8A bis 8D)

- In Bereitstellungsstation **2** und Eingangsstation **3,** frontseitige erste Zone **31:**
   Das neue, dritte Gebinde **93** ist komplett in die erste Zone **31** eingeschoben und wird von erster Fördereinrichtung **34** getragen. Auf der Auflage **20** verbleiben die vom Greifer **21** zurückgehaltene und zur Entsorgung bestimmte Umhüllung **99** sowie das in Ausgangsposition zurückbewegte Vorschubmittel **22.**
- Im Containment **4,** frontseitige erste Zone **41:**
   Die im Deckel **51** integrierte Dekontaminationsvorrichtung **7** kann abgeschaltet werden; die Behandlung ist abgeschlossen. Mit Abschwenken des Auslegers **50** der Apparatur **5** wird der Deckel **51** von der ersten Passage **47** abgehoben. Zugleich wird der Werkzeugkopf **61** des Manipulators **6** hin zur ersten Passage **47** bewegt.
- Im Containment **4,** rückseitige zweite Zone **42:**
   Mit Umschwenken des Auslegers **50** der Apparatur **5** wird der Deckel **51** der zweiten Passage **48** angenähert.

### Ablauf in Prozessphase 7 (Figuren 9A bis 9D)

- Im Containment **4,** frontseitige erste Zone **41:**
   Der Deckel **51** ist von der ersten Passage **47** entfernt und das vom Manipulator **6** geführte Werkzeug wird der Abdeckung **98** des zweiten Gebindes **92** angenähert.
- Im Containment **4,** rückseitige zweite Zone **42** und Eingangsstation **3,** rückseitige zweite Zone **32:**
   Mit weiterem Schwenken des Auslegers **50** der Apparatur **5** gelangt der Deckel **51** mit der darin integrierten Dekontaminationsvorrichtung **7** auf die zweite Passage **48.** Anschliessend wird mit Abschwenken der zweiten Fördereinrichtung **35** das Behältnis **94** des verarbeiteten ersten Gebindes **91** von der zweiten Passage **48** entfernt und auf die erste Fördereinrichtung **34** zum Abtransport nach ausserhalb der Schleusenanordnung **1** aufgesetzt.

### Ablauf in Prozessphase 8 (Figuren 10A bis 10D)

- In Bereitstellungsstation **2:**
   Wie in Prozessphase 1 gemäss Startposition wird ein nächstes neues, drittes Gebinde **93,** wiederum mit noch verschlossener Umhüllung **99** auf der Auflage **20,** vor dem Zugang **37,** positioniert.
- Im Containment **4,** frontseitige erste Zone **41:**
   Mit dem am Werkzeugkopf **61** des Manipulators **6** installierten Werkzeug wird die Abdeckung **98** des zweiten Gebindes **92,** welches an der ersten Passage **47** angedrückt ist, z.B. durch Aufschneiden geöffnet und der dabei entstehende Schnittabfall entfernt, so dass Zugang zum Durchlass **95** in das Behältnis **94** geschaffen ist.
- In Eingangsstation **3,** rückseitige zweite Zone **32:**
   Ein auf der Bereitstellungsstation **2** von der Umhüllung **99** befreites, weiteres erstes Gebinde **91** mit intakter Abdeckung **98** wurde quer auf die erste Fördereinrichtung **34** transferiert und inzwischen auch auf die zweite Fördereinrichtung **35** geladen.

### Ablauf in Prozessphase 9 (Figuren 11A bis 11D)

- In Bereitstellungsstation **2:**
   Wie in Prozessphase 3 gemäss Figur 5A wird mittels des Werkzeugs **23** die bis anhin verschlossene Umhüllung **99** geöffnet.
- Im Containment **4,** frontseitige erste Zone **41:**
   Wie in Prozessphase 3 gemäss Figur 5B wird mit dem vom Manipulator **6** geführten Werkzeug, bei offener Abdeckung **98,** die optional vorhandene Zwischenlage **97** aus dem zweiten Gebinde **92,** das an der ersten Passage **47** angedrückt ist, entnommen.
- In Eingangsstation **3,** rückseitige zweite Zone **32:**
   o Durch Aufschwenken der zweiten Fördereinrichtung **35** wird das weitere erste, quer transferierte Gebinde **91** mit der Abdeckung **98** an die zweite Passage **48** angedrückt.
   ∘ Somit wird wieder, wie bereits in Prozessphase 3 gemäss Figur 5A , an der ersten Passage **47** entstanden, jetzt an der zweiten Passage **48,** ein zwischen Deckel **51,** Abdeckung **98** des zweiten Gebindes **92,** der die zweite Passage **48** umrandenden Dichtung **480** und möglichen freiliegenden Wandungsflächen ein Umfeld **8** samt davon eingeschlossenes und in der nächsten Prozessphase 10 zu dekontaminierendes Gasvolumen gebildet.

### Ablauf in Prozessphase 10 (Figuren 12A bis 12D)

- In Bereitstellungsstation **2:**
   Hier wiederholt sich die Situation von Prozessphase 4 gemäss Figur 6A, allerdings nun mit dem nächsten neuen, dritten Gebinde **93.**
- Im Containment **4,** frontseitige erste Zone **41:**
   Hier wiederholt sich die Situation von Prozessphase 4 gemäss Figur 6B, allerdings nun mit dem zweiten Gebinde **92,** welches jetzt an der ersten Passage **47** angedrückt ist.
- In Eingangsstation **3,** rückseitige zweite Zone **32,** und Containment **4,** rückseitige zweite Zone **42:**
   Das in der vorherigen Prozessphase 9 gemäss Figur 11B gebildete Umfeld **8** mit darin eingeschlossenem Gasvolumen wird mittels der aktivierten Dekontaminationsvorrichtung **7** dekontaminiert.

### Ablauf in Prozessphase 11 (Figuren 13A bis 13D)

- In Bereitstellungsstation **2:**
   Hier wiederholt sich die Situation von Prozessphase 5 gemäss Figur 7A, allerdings nun mit dem nächsten neuen, dritten Gebinde **93.**
- Im Containment **4,** frontseitige erste Zone **41:**
   Hier wiederholt sich die Situation von Prozessphase 5 gemäss Figur 7B, allerdings wird nun in das Behältnis **94** des zweiten Gebindes **92,** welches jetzt an der ersten Passage **47** angedrückt ist, ein leeres Nest **96** aus der Prozesskammer **43** zurückgeführt.
- In Eingangsstation **3,** rückseitige zweite Zone **32,** und Containment **4,** rückseitige zweite Zone **42:**
   Die Dekontamination läuft weiter.

### Ablauf in Prozessphase 12 (Figuren 14A bis 14D)

- In Bereitstellungsstation **2:**
   Hier wiederholt sich die Situation von Prozessphase 6 gemäss Figur 8A, allerdings nun mit dem nächsten neuen, dritten Gebinde **93.**
- Im Containment **4,** frontseitige erste Zone **41:**
   Hier wiederholt sich die Situation von Prozessphase 6 gemäss Figur 8B, allerdings nun mit dem zweiten Gebinde **92** und an der ersten Passage **47,** der mit Umschwenken des Auslegers **50** der Apparatur **5** der Deckel **51** angenähert wird.
- In Eingangsstation **3,** rückseitige zweite Zone **32,** und Containment **4,** rückseitige zweite Zone **42:**
   Die Dekontamination im Umfeld **8** an der zweiten Passage **48** mit dem weiteren ersten, quer transferierten Gebinde **91** ist beendet. Der Deckel **51** mit der Dekontaminationsvorrichtung **7** sind weggeschwenkt und der Manipulator **6** mit den am Werkzeugkopf **61** installierten Werkzeugen ist an die zweite Passage **48** herangeschwenkt.

### Ablauf in Prozessphase 13 (Figuren 15A bis 15D)

- In Eingangsstation **3,** frontseitige erste Zone **31** und Containment **4,** frontseitige erste Zone **41:**
   Zuerst schwenkt die Apparatur **5** den Deckel **51** auf die erste Passage **47,** diese verschliessend. Anschliessend wird durch Abschwenken der zweiten Fördereinrichtung **35** das Behältnis **94** des verarbeiteten zweiten Gebindes **92** mit dem rückgeführten Nest **96** auf die erste Fördereinrichtung **34** zwecks querverlaufendem Abtransport nach ausserhalb der Schleusenanordnung **1** geladen. Somit wird eine Kollision mit dem nächsten neuen, dritten Gebinde **93** vermieden, welches bereits auf der ersten Fördereinrichtung **34** steht.
- In Eingangsstation **3,** rückseitige zweite Zone **32** und Containment **4,** rückseitige zweite Zone **42:**
   Hier wiederholt sich die Situation wie in Prozessphase 1 gemäss Figur 3B, mit einem Werkzeug am Manipulator **6** wird die Abdeckung **98** geöffnet, allerdings jetzt des ursprünglich quer auf die erste Fördereinrichtung **34** transferierten neuen ersten Gebindes **91.**

### Figuren 16A und 16B

Bei dieser alternativen Anordnung ist die Dekontaminationsvorrichtung **7** nicht, wie bis anhin, im Deckel **51** integriert, sondern das Emissionselement der Dekontaminationsvorrichtung **7,** z.B. eine Vernebelungsdüse zum Eintrag eines H2 O2 -Aerosols, sitzt nicht im Deckel **51,** sondern ausserhalb in einer Halterung **70.** Diese Halterung **70** kann in einfachster Gestalt von einer Abkantung der zwischen Eingangsstation **3** Containment **4** sich erstreckenden Trennwand **40** gebildet sein. Die dem Umfeld **8** zugewandte Oberfläche der Halterung **70** ist dann Bestandteil des im Produktionsprozess zu dekontaminierenden eingeschlossenen Gasvolumens und Umfeld **8.** Unverändert wird der Deckel **51** von einem schwenkbaren Ausleger **50** einer in der Prozesskammer **43** stationierten Apparatur **5** getragen.

## Patentansprüche

1. Verfahren zum Transfer von Artikeln (960) eines Gebindes (9) in eine Prozesskammer (43) eines Containments (4), wobei das Containment (4) über zumindest eine Passage (47), vorzugsweise zwei Passagen (47, 48), zum Transfer verfügt, wobei das Gebinde (9) mit einer Fördereinrichtung (35) an die zumindest eine Passage (47) herangeschwenkt wird, **dadurch gekennzeichnet, dass** die Fördereinrichtung (35) eine Gabel (352) und einen schwenkbaren Träger (353) umfasst, wobei der Träger (353) an der Gabel (352) aufgehängt ist, und der Träger (353) das Gebinde (9) unterfasst und die Fördereinrichtung (35) das Gebinde (9) an die zumindest eine Passage (47) schwenkt.

2. Verfahren nach Anspruch 1, **dadurch gekennzeichnet, dass** der Träger (353) kardanisch an der Gabel (352) aufgehängt ist, und der Träger (353) das Gebinde (9) unterfasst und die Fördereinrichtung (35) das Gebinde (9) kardanisch aufgehängt an die zumindest eine Passage (47) schwenkt.

3. Verfahren nach einem der vorangehenden Ansprüche, **dadurch gekennzeichnet, dass** die Gabel (352) seitlich in das Gebinde (9) einfährt und das Gebinde (9) anschließend an die Passage (47) schwenkt.

4. Transfervorrichtung, zum Transfer von Artikeln (960) eines Gebindes (9) in eine Prozesskammer (43) eines Containments (4), wobei das Containment (4) über zumindest eine Passage (47), vorzugsweise zwei Passagen (47, 48), zum Transfer verfügt, wobei die Transfervorrichtung eine Fördereinrichtung (35) umfasst, **dadurch gekennzeichnet, dass** die Fördereinrichtung (35) eine Gabel (352) und einen schwenkbaren Träger (353) umfasst, wobei der Träger (353) an der Gabel (352) aufgehängt ist, wobei die Fördereinrichtung (35) eine Schwenkvorrichtung umfasst, um das Gebinde (9) an die zumindest eine Passage (47) zu schwenken.

5. Transfervorrichtung nach Anspruch 4, **dadurch gekennzeichnet, dass** das Gebinde (9) mit einer Fördereinrichtung (35) an die zumindest eine Passage (47) herangeschwenkt wird, wobei die Fördereinrichtung (35) eine Gabel (352) und einen schwenkbaren Träger (353) umfasst, wobei der Träger (353) an der Gabel (352) aufgehängt ist, wobei der Träger (353) kardanisch an der Gabel (352) aufgehängt ist.

6. Transfervorrichtung nach einem der vorangehenden Ansprüche, **dadurch gekennzeichnet, dass** die zumindest eine Passage (47) eine Dichtung aufweist.

7. Transfervorrichtung nach einem der vorangehenden Ansprüche, **dadurch gekennzeichnet, dass** der Träger (353) zwei parallele Tragarme hat und/oder dass der Träger (353) an wenigstens einer von vier Seiten geöffnet ist.

8. Transfervorrichtung nach einem der vorangehenden Ansprüche, **dadurch gekennzeichnet, dass** die Gabel (352) angetrieben ist.

9. Transfervorrichtung nach einem der vorangehenden Ansprüche, **dadurch gekennzeichnet, dass** der Träger (353) an zwei Punkten an der Gabel (352) aufgehängt ist und eine Drehachse (D3) durch diese Punkte verläuft, wobei die Drehachse (D3) rechtwinklig zu einer Längsachse von Zinken der Gabel (352) verläuft.

## Claims

1. Method for transferring articles (960) of a container (9) into a process chamber (43) of a containment (4), wherein the containment (4) has at least one passage (47), preferably two passages (47, 48), for transfer, wherein the container (9) is pivoted towards the at least one passage (47) by means of a conveying device (35), **characterized in that** the conveying device (35) comprises a fork (352) and a pivotable carrier (353), wherein the carrier (353) is suspended from the fork (352), and the carrier (353) grips the container (9) underneath and the conveying device (35) pivots the container (9) to the at least one passage (47).

2. Method according to claim 1**, characterized in that** the carrier (353) is gimbal-mounted on the fork (352), and the carrier (353) grips the container (9) underneath and the conveying device (35) pivots the container (9) to the at least one passage (47) in a gimballed manner.

3. Method according to one of the preceding claims, **characterized in that** the fork (352) moves laterally into the container (9) and then pivots the container (9) to the passage (47).

4. Transfer device for transferring articles (960) of a container (9) into a process chamber (43) of a containment (4), wherein the containment (4) has at least one passage (47), preferably two passages (47, 48), for transfer, wherein the transfer device comprises a conveying device (35), **characterized in that** the conveying device (35) comprises a fork (352) and a pivotable carrier (353), wherein the carrier (353) is suspended from the fork (352), wherein the conveying device (35) comprises a pivoting device for pivoting the container (9) to the at least one passage (47).

5. Transfer device according to claim 4 **characterized in that** in that the container (9) is pivoted towards the at least one passage (47) by a conveying device (35), wherein the conveying device (35) comprises a fork (352) and a pivotable carrier (353), wherein the carrier (353) is suspended on the fork (352), wherein the carrier (353) is gimbal-mounted on the fork (352).

6. Transfer device according to one of the preceding claims, **characterized in that** the at least one passage (47) has a seal.

7. Transfer device according to one of the preceding claims, **characterized in that** the carrier (353) has two parallel support arms and/or **in that** the carrier (353) is open on at least one of four sides.

8. Transfer device according to one of the preceding claims, **characterized in that** the fork (352) is driven.

9. Transfer device according to one of the preceding claims, **characterized in that** the carrier (353) is suspended from the fork (352) at two points and an axis of rotation (D3) extends through these points, wherein the axis of rotation (D3) extends at right angles to a longitudinal axis of tines of the fork (352).

## Revendications

1. Procédé pour le transfert d'articles (960) d'un contenant (9) à une chambre de traitement (43) d'un confinement (4), dans lequel le confinement (4) dispose d'au moins un passage (47), de préférence de deux passages (47, 48), pour le transfert, lequel le contenant (9) est basculé avec une installation de transport (35) vers l'au moins un passage (47), **caractérisé en ce que** l'installation de transport (35) comprenant une fourche (352) et un support basculant (353), lequel support (353) est suspendu à la fourche (352), et le support (353) saisissant le contenant (9) par dessous et l'installation de transport (35) faisant basculer le contenant (9) vers l'au moins un passage (47).

2. Procédé selon la revendication 1**, caractérisé en ce que** lequel support (353) est suspendu à la fourche (352) par un cardan, et le support (353) saisissant le contenant (9) par dessous et l'installation de transport (35) faisant basculer le contenant (9) vers l'au moins un passage (47) avec une suspension à la Cardan.

3. Procédé selon l'une des revendications précédentes, **caractérisé en ce que** la fourche (352) entre dans le contenant (9) par le côté et fait ensuite basculer le contenant (9) vers le passage (47).

4. Dispositif de transfert pour le transfert d'articles (960) d'un contenant (9) vers une chambre de traitement (43) d'un confinement (4), dans lequel le confinement (4) dispose d'au moins un passage (47), de préférence de deux passages (47, 48), pour le transfert, dans lequel le dispositif de transfert comprend une installation de transport (35), **caractérisé en ce que** l'installation de transport (35) comprend une fourche (352) et un support basculant (353), lequel support (353) est suspendu à la fourche (352), l'installation de transport (35) comprenant un dispositif de basculement pour faire basculer le contenant (9) vers l'au moins un passage (47).

5. Dispositif selon la revendication 4**, caractérisé en ce que** le contenant (9) est basculé avec une installation de transport (35) vers l'au moins un passage (47), l'installation de transport (35) comprenant une fourche (352) et un support basculant (353), lequel support (353) est suspendu à la fourche (352), dans lequel le support (353) est suspendu à la fourche (352) par un cardan.

6. Dispositif de transfert selon l'une des revendications précédentes, **caractérisé en ce que** l'au moins un passage (47) comporte une garniture d'étanchéité.

7. Dispositif de transfert selon l'une des revendications précédentes, **caractérisé en ce que** le support (353) possède deux bras de support parallèles et/ou **en ce que** le support (353) est ouvert sur au moins un de quatre côtés.

8. Dispositif de transfert selon l'une des revendications précédentes, **caractérisé en ce que** la fourche (352) est entrainée.

9. Dispositif de transfert selon l'une des revendications précédentes, **caractérisé en ce que** le support (353) est suspendu à la fourche (352) en deux points et un axe de rotation (D3) passe par ces points, l'axe de rotation (D3) formant un angle droit avec un axe longitudinal de dents de la fourche (352).
